# EUROPEAN PATENT SPECIFICATION

(11) **EP 4 294 486 B1**
(45) Date of publication and mention of the grant of the patent: **22.10.2025**
(21) Application number: 22757066.0
(22) Date of filing: 21.02.2022
(51) Int. Cl.: A61M 16/00, A61M 16/10, A61M 16/20

(54) **VENTILATOR SYSTEM**
VENTILATORSYSTEM
SYSTÈME DE VENTILATEUR

(30) Priority: 22.02.2021 US 202163151913 P
(43) Date of publication of application: 27.12.2023
(73) Proprietor: Ventis Medical, Inc., Princeton, NJ 08540 (US)
(72) Inventor: BARENBOYM, Michael, Miami Beach, FL 33139 (US); MEIER, Giovanni, Madison, CT 06443 (US); LAUB, Glenn, W., Princeton, NJ 08540 (US); LAUB, David, G., Seattle, WA 98122 (US); LAUB, Taylor, R., New York, NY 10010 (US); LAUB, Karen, Princeton, NJ 08540 (US); JACOBSON, Steve, Purchase, NY 10577 (US)
(74) Representative: Marks & Clerk LLP
(86) International application number: PCT/US2022/017146
(87) International publication number: WO 2022/178349

(56) References cited:
- EP-A1- 3 305 355
- US-A1- 2010 307 498
- US-A1- 2011 197 884
- US-A1- 2016 114 121
- US-A1- 2020 188 616
- US-A1- 2020 368 468

## Description

The present invention generally relates to a ventilator and ventilator system for assistance with breathing and, more particularly, to an enclosure system and breathing circuit of a ventilator system.

US 2010/0307498 A1 relates to a flow generator configured to provide a flow of breathable gas. A chassis assembly includes an upper chassis and a lower chassis. A seal includes a blower receptacle sealing portion configured to seal a blower receptacle, which is configured to receive a blower that generates the flow of breathable gas. A flow sensor sealing portion seals a flow sensor provided in the chassis assembly, and measures the flow of breathable gas. The seal and the chassis assembly define a flow path from an inlet of the chassis assembly to the blower receptacle of the chassis assembly to an outlet of the chassis assembly.

EP 3305355 A1 relates to flow generator that includes a housing, a blower structured to generate a flow of pressurized breathable air, and a suspension device to support the blower within the housing and provide a pressure seal between low and high pressure sides of the blower. The suspension device includes a bellows-like portion provided along the perimeter of the blower to absorb shock applied at least radially to the blower and one or more cones provided along upper and/or lower sides of the blower to absorb shock applied at least axially to the blower.

US 2011/197884 A1 relates to a ventilator. A porting system has a plurality of sensors structured to monitor a number of parameters with respect to the flow of gas, and a number of porting blocks is used to reconfigure the ventilator so that it operates as a single-limb or dual limb ventilator. In the single-limb configuration, an active or passive exhaust assembly can be provided proximate to the patient.

US 2016/114121 A1 relates to a respiratory treatment apparatus to provide a flow of breathable gas to a patient, which includes a breathable air outlet, an outside air inlet, and an pneumatic block module. The pneumatic block module includes a volute assembly including an inlet air passage, a mount for a blower and an outlet air passage. The blower is mounted in the mount such that an impeller of the blower is in a flow passage connecting the inlet air passage and the outlet air passage. The inlet air passage is in fluid communication with the outside air inlet and the outlet air passage is in fluid communication with the air outlet.

### SUMMARY

The invention is defined in claim 1 and its appended dependent claims. The present invention is directed to a ventilator including: a housing having a front panel, and a rear panel opposite the front panel and configured to couple to the front panel; a receptacle coupled to the rear panel of the housing, the receptacle having a recess; a sealing element disposed within the recess, the sealing element having an inlet and an aperture; and a blower having a blower outlet and a blower inlet, the blower inlet coupled to the sealing element such that the blower inlet at least partially abuts the aperture. The receptacle and the sealing element comprise an airflow pathway configured to allow for flow of air from the inlet of the sealing element into the blower inlet and out of the blower outlet. The blower outlet and the inlet are coupled to a sensor, the sensor configured to receive air from the blower outlet and direct the air to the inlet.

In some embodiments, the blower is configured to vent gas when the gas reaches a predetermined amount. The gas may be oxygen, and the predetermined amount may be at least 25%.

In some embodiments, the blower is configured to continuously vent gas to keep the gas at or below a predetermined level. The gas may be oxygen, and the predetermined level may be at least 25%.

In some embodiments, the ventilator further includes a gap between the blower inlet and the receptacle to allow air to flow into blower inlet. The receptacle may be integrally formed with the rear panel of the housing. The receptacle may be coupled to the rear panel of the housing.

In some embodiments, the recess includes a recessed lip and the sealing element is surrounded by the recess lip. The recess may be sized and shaped to receive the sealing element.

In some embodiments, the rear panel of the housing includes a cutout, and the receptacle is disposed within the cutout.

In some embodiments, the aperture of the sealing element is sized and shaped to receive at least a portion of the blower inlet.

In some embodiments, the sealing element is an additively manufactured enclosure.

In some embodiments, the ventilator further includes a securing element coupled to the receptacle, the securing element configured to receive the blower and secure the blower and sealing element within the recess of the receptacle.

In some embodiments, the blower, the sealing element, and the receptacle are coupled together to form a substantially airtight seal.

In some embodiments, the ventilator further includes one or more ports disposed on the housing and a port plate configured to at least partially cover the one or more ports, wherein the port plate is removable from the housing.

In some embodiments, the ventilator further includes one or more ports disposed on the housing and a door configured to at least partially cover the one or more ports, wherein the door is hingedly coupled to the housing and includes at least one of a plug and connection channel.

In some embodiments, the ventilator further includes a breathing circuit coupled to the sealing element, the breathing circuit having an exhale valve configured to open and closed based on air flow generated by the blower.

### BRIEF DESCRIPTION OF THE DRAWINGS

The following detailed description of embodiments of the ventilator system will be better understood when read in conjunction with the appended drawings of exemplary embodiments. It should be understood, however, that the invention is defined by the claims and is not limited to the precise arrangements and instrumentalities shown.
Fig. 1A is a top plan view of a ventilator system having a ventilator, breathing circuit, and patient interface in accordance with an exemplary embodiment of the present invention
Fig. 1B is a schematic diagram of the ventilator system of Fig. 1A;
Fig. 2 is a top perspective view of a ventilator in accordance with an exemplary embodiment of the present invention;
Fig. 3 is a bottom view of the ventilator of Fig. 2;
Fig. 4 is an exploded view of the ventilator of Fig. 2;
Fig. 5 is an exploded view of the ventilator of Fig. 4;
Fig. 6 is an exploded view of the ventilator of Fig. 5 with an enclosure in position;
Fig. 7 is a bottom perspective view of the enclosure of Fig. 6;
Fig. 8 is a bottom perspective view of a blower and the enclosure of Fig. 7;
Fig. 9 is a cross-section view of Fig. 8;
Fig. 10 is an exploded view of a ventilator in accordance with an exemplary embodiment of the present invention;
Fig. 11 is an exploded view of the ventilator of Fig. 10;
Fig. 12 is an exploded view of the ventilator of Fig. 10 with the fan assembly in position;
Fig. 13 is a cross-sectional perspective side view of the ventilator of Fig. 6;
Fig. 14 is a bottom view of the ventilator of Fig. 2;
Fig. 15 is a top view of the interior of the ventilator of Fig. 13;
Fig. 16A is a top view of the ventilator of Fig. 15;
Fig. 16B is a top view of the ventilator of Fig. 16A illustrating the airflow;
Fig. 17A is a perspective partial view of the ventilator of Fig. 2;
Fig. 17B is an cross-sectional top view of the ventilator of Fig. 17A illustrating the airflow;
Fig. 18 is zoomed in view of the ventilator of Fig. 12;
Fig. 19 is a schematic diagram of the ventilator system of Fig. 1A;
Fig. 20 is a schematic diagram of a ventilator unit and breathing circuit in accordance with an exemplary embodiment of the present invention; and
Fig. 21 is a top rear perspective view of the ventilator of Fig. 2.

### DETAILED DESCRIPTION

Ventilators have been commonly used to provide treatment to individuals in respiratory distress. For example, ventilators are used in medical and hospital settings to treat individuals who are unable to breath on their own or who receive an inadequate amount of oxygen to their lungs. Current ventilators are bulky and require many wires and large towers located proximate to the patient. These ventilators are not easily portable and do not allow for efficient disassembly if an issue arose. Further, current ventilators may leak air, which can result in inefficiency in delivering gas to the patient, and/or may cause buildup of air or gas (e.g., oxygen) within the blower, which can be potentially dangerous (e.g., fire hazard).

Exemplary embodiments of the present invention provide a ventilator system, generally designated 100. In use, ventilator system 100 may be used to provide assistance with breathing for the treatment of patients in a medical setting, such as the intensive care unit (ICU) of a hospital or a medical clinic. Ventilator system 100 may also be used in other settings such as an ambulance, ambulatory center, in/out- patient centers, nursing homes/long term care facilities, and mobile clinics that can go to a patient directly. In some examples, ventilator system 100 is portable to allow for use in different environments. For example, ventilator system 100 may be easily transportable to be used in mobile settings (e.g., an ambulance).

In some examples, ventilator system 100 allows for rapid initiation of emergency ventilation to a patient in respiratory distress. Ventilator system 100 may be configured to provide rapid, emergency ventilation to a patient with minimal to no leakage of waste of air. Ventilator system 100 may provide an efficient system for providing ventilation to a patient. As discussed in further detail below, ventilator system 100 may, among other benefits, provide a modular fan assembly that allows for components of the fan assembly to be easily replaced while generating air or gas with minimal to no leakage of air and without causing buildup of air or gas within the fan assembly.

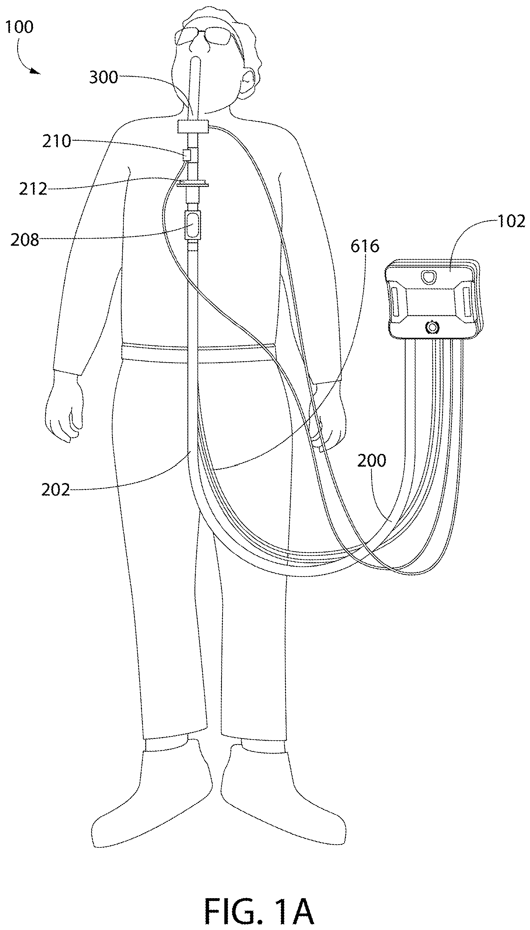

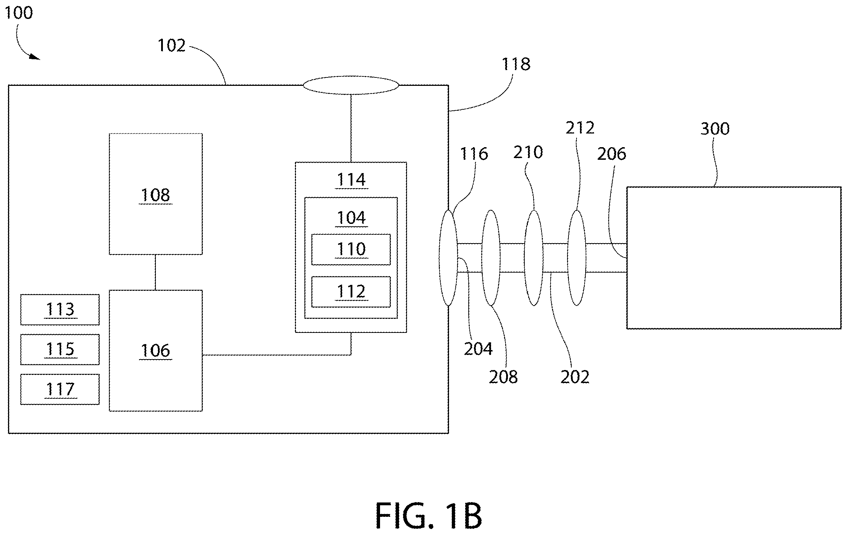

Referring to Figs. 1A-1B, ventilator system 100 may include a medical device, such as a ventilator, to assist patients in respiratory distress or acute respiratory failure. In some examples, ventilator system 100 includes ventilator 102 for generating airflow for ventilation, breathing system or breathing circuit 200 for controlling ventilation to a patient, and patient interface 300 for delivering ventilation to the patient. Breathing circuit 200 and patient interface 300 may be coupled to ventilator 102. In some examples, breathing circuit 200 is configured to couple ventilator 102 to patient interface 300. For example, breathing circuit 200 may be disposed between ventilator 102 and patient interface 300 to provide an air pathway from ventilator 102 to patient interface 300.

In practice, ventilator 102 may output air, and breathing circuit 200 may provide a controlled pathway for the air to flow from ventilator 102 to patient interface 300. For example, breathing circuit 200 may include a tube (e.g., breathing tube) with one or more valves to control the flow of air from ventilator 102 to patient interface 300. Patient interface 300 may include a device (e.g., mask or nasal cannula) coupled to a patient to provide the air from ventilator 102 via breathing circuit 200.

Ventilator 102 may serve as the control hub or control system of ventilator system 100. Ventilator 102 may be configured to provide mechanical ventilation to a patient under respiratory failure through breathing circuit 200. Ventilator 102 may provide the necessary gas flow or airflow, which may be directed through breathing circuit 200 to patient interface 300, which is coupled to the face of a patient (e.g., the mouth and/or nose of the patient). Ventilator 102 may include blower 104, control system 106 and power supply 108. Breathing circuit 200 may include tube 202 which may be coupled to ventilator 102 at first end 204 and coupled to patient interface 300 at second end 206. Patient interface 300 may be a device that is secured to the face of a patient. For example, patient interface 300 may be a bag valve mask, respirator, or an endotracheal (ET) tube used for intubation.

In some examples, ventilator 102 is used to provide assistance to a patient in respiratory distress. Ventilator 102 may be a pump, pneumatic system, or any other type of device configured to provide air to a patient and/or assist a patient with respiration. Ventilator 102 may be configured to provide different modes of ventilation to a patient. For example, ventilator 102 may be configured to provide assist-control ventilation, volume-controlled ventilation, pressure support, pressure-controlled ventilation, pressure regulated volume control, positive end expiatory pressure, synchronized intermittent-mandatory ventilation, and/or manual ventilation. Ventilator 102 may be used instead of a bag valve device, an emergency transport ventilator, or any other modes or devices for providing ventilation to a patient.

Referring to Figs. 1A-4, ventilator 102 may include housing 132, blower 104, control system 106, and power supply 108. Housing 132 of ventilator 102 may house and protect the components disposed within ventilator 102. In some examples, housing 132 is comprised of two halves coupled together, such as front panel 129 and rear panel 131 coupled together via screws, adhesive, magnets, or any other coupling mechanism. Ventilator 102 may be lightweight to be easily portable. For example, housing 132 of ventilator 102 may be made of a lightweight polymer to allow for easy transportation. In some examples, housing 132 is manufactured via injection molding. Housing 132 may be manufactured using a computer numerical control (CNC) machine and/or via additive manufacturing, such as 3D printing. Housing 132 may have a length of approximately 165 mm, a width of approximately 202 mm, and a thickness of approximately 58 mm. However, housing 132 may have a length of approximately 50 mm to approximately 250 mm, approximately 75 mm to approximately 225 mm, approximately 100 mm to approximately 200 mm, or approximately 125 mm to approximately 175 mm, a width of approximately 100 mm to approximately 300 mm, approximately 125 mm to approximately 275 mm, approximately 150 mm to approximately 250 mm, or approximately 175 to approximately 225 mm, and a thickness of approximately 25 mm to 200 mm, approximately 50 mm to approximately 175 mm, or approximately 75 mm to approximately 150 mm.

In some examples, housing 132 is lightweight to allow for easy portability of ventilator 102. For example, housing 132 may be comprised a lightweight yet durable material to allow for easy transportation of ventilator 102 while still providing protection for the internal components of ventilator 102. In some examples, housing 132 is comprised of one or more of acrylonitrile butadiene styrene (ABS), polyoxymethylene (POM), aliphatic polyamides (PPA), polycarbonate (PC), polyphenylsulfone (PPSU), polyetherimide (PEI), and polypropylene (PP). Housing 132 may be comprised of a lightweight, but durable material to allow for repeated use in harsh environments while still providing portability. For example, housing 132 may be comprised of ABS to provide portability and ensure that the components disposed within housing 132 remain secured and undamaged during use and/or transportation. In some examples, housing 132 of ventilator 102 is substantially rectangular shaped to allow for easy storage. However, housing 132 may be square, circular, triangle, octagonal, or any other shape desired. In some examples, housing 132 includes sidewalls 130. In a preferred example, housing 132 includes four sidewalls 130 to define a substantially rectangular shape of ventilator 102. In some examples, housing 132 has rounded corners and beveled edges to allow for a more ergonomic shape and to prevent injury to a user.

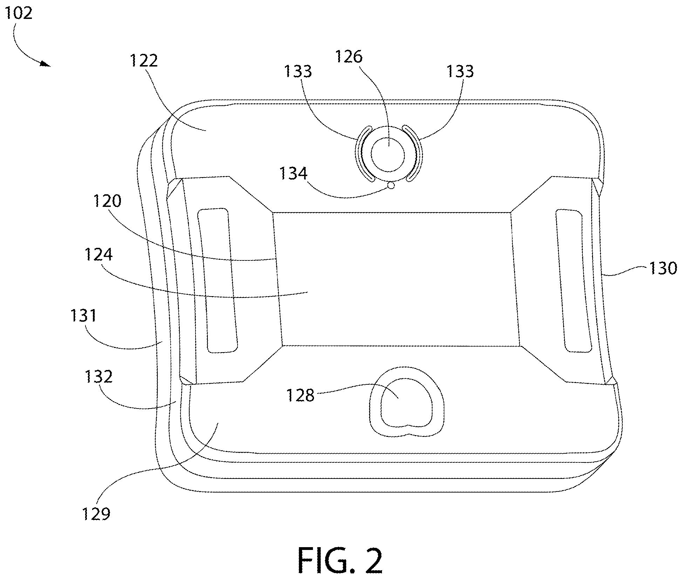

Referring to Figs. 2-3, housing 132 may include top surface 122 and bottom surface 139. In some examples, top surface 122 is parallel to bottom surface 139. Top surface 122 may be coupled to bottom surface 139 via sidewalls 130. In some examples, housing 132 includes four sidewalls, each configured to couple top surface 122 to bottom surface 139. Sidewalls 130 and top surface 122 and/or bottom surface 139 may be integrally formed. For example, sidewalls 130 and top surface 122 or bottom surface 139 may form a unitary piece.

Housing 132 may include cutout 120 disposed on top surface 122 of housing 132. Cutout 120 may be sized and shaped to receive user interface 124. User interface 124 may be a display device, which may be disposed within cutout 120 and may be configured to receive input from a user. For example, user interface 124 may be a liquid crystal display (LCD), a light emitting diode (LED) display, an organic LED display, or any other type of display. In some examples, user interface 124 is a graphical user interface. For example, user interface 124 may be a touch screen configured to receive inputs from a user and transmits the inputs to control system 106. Further, user interface 124 may be used to display information about a patient using ventilator 102. For example, user interface 124 may provide (e.g., display) an indication of the respiratory status of a patient coupled to ventilator 102 via patient interface 300. In some examples, user interface 124 may display various settings, parameters, and/or functionalities of the components disposed within ventilator 102. For example, user interface 124 may display the peak inspiratory pressure (PIP), tidal volume (TV), respiratory rate (RR), positive end expiratory pressure (PEEP), inspiratory to expiatory ratio (I:E ratio), ventilation mode, peak flow, and sensitivity. User interface 124 may be coupled to control system 106 and may be configured to control various components of ventilator system 100. For example, a user may interact with user interface 124 to change parameters of blower 104. In some examples, user interface 124 is configured to display instructions to the user. For example, user interface 124 may provide instructions to a user for correcting an error to ventilator 102. In some examples, user interface 124 is configured to display a video or graphics to a user to instruct them on how to fix or address an error associated with ventilator 102.

In some examples, a user interacts with user interface 124 to change various modes and/or parameters of ventilator 102. For example, user interface 124 may provide an option for adjusting the PEEP, the PIP, the tidal volume, the I:E ratio, or other parameters. In some examples, ventilator 102 includes beacon or indicator 134 to provide a status of ventilator system 100. Indicator 134 may provide the status of ventilator system 100 and/or ventilator 102. For example, indicator 134 may indicate whether ventilator 102 is damaged, inoperable, and/or functionally properly. Indicator 134 may be an LED, and control system 106 may transmit a status to indicator 134 causing indicator 134 to illuminate a specific color and/or flash at a specific frequency. However, indicator 134 may be a transmitter configured to transmit an outgoing signal. In some examples, indicator 134 is configured to continuously transmit an outgoing signal regarding the status of ventilator 102. For example, indicator 134 may be configured to continuously transmit a signal without being requested to transmit a signal. Indicator 134 may transmit a signal indicating all components of ventilator 102 are functioning correctly.

In some examples, indicator 134 continuously transmits a signal until an error occurs, which interrupts the signal transmission resulting in indicator 134 no longer transmitting a signal. A user may check a receiver to determine whether indicator 134 is transmitting a signal and whether an error has occurred based on the transmission ceasing. In other examples, indicator 134 is configured to transmit a first signal when ventilator 102 is functioning correctly without significant errors and is configured to transmit a second signal when an error occurs. The first signal may be different than the second signal. Indicator 134 may transmit a signal wirelessly via radio frequency, Wi-Fi, cellular signal, Bluetooth, near field communication, or any other type of wireless modality.

In some examples, housing 132 may further include indicators 133. Indicator 133 may indicate the status of ventilator 102 and may be used to provide alerts to the user regarding an alarm condition. For example, indicator 133 being green in color may indicate normal operation of ventilator 102. However, indicator 133 flashing amber, red, yellow, or orange may indicate a malfunction or error with ventilator 102. In some examples, the degree of flashing of indicator 133 indicates the severity of the error. Indicator 134 may also indicate the battery status associated with power supply 108. For example, indicator 134 being green may indicate that the battery of ventilator 102 is fully charged. Indicator 134 being other colors, such as red, orange, yellow, amber, and/or flashing may indicate a malfunction or power level of the battery.

Ventilator 102 may include one or more buttons that control ventilator system 100. For example, ventilator 102 may include buttons 126 and 128, which control the power status and functions of ventilator 102. In some examples, button 126 is a power button (e.g., ON/OFF button) to control the power status of ventilator 102. For example, a user may press button 126 to power on ventilator 102. Button 128 may be a manual breath button, which delivers a single breath at a predetermined tidal volume to a patient. In some examples, button 128 may need to be pressed for a predetermined amount of time before ventilator 102 delivers a single breath to the patient.

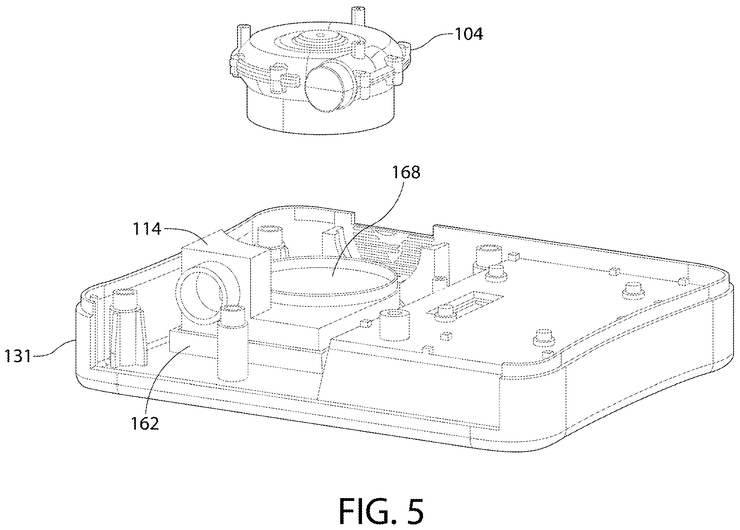

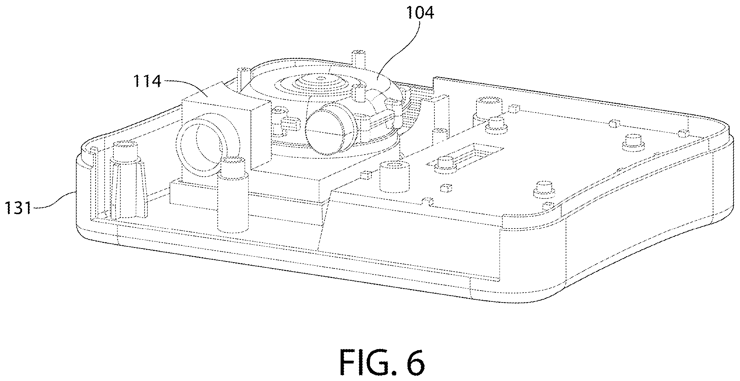

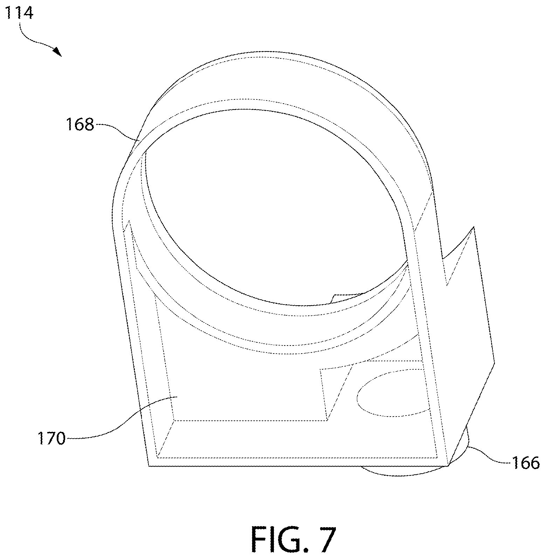

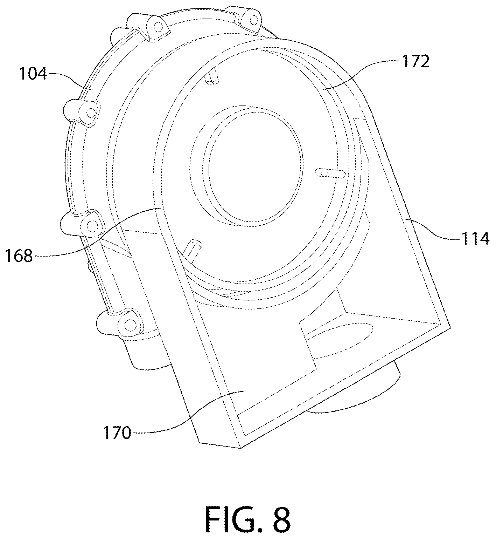

Referring to Figs. 1A-1B and 4-9, ventilator 102 may include blower or pneumatic system 104, which may include motor 110 and fan 112. Motor 110 may be coupled to fan 112 and motor 110 may be configured to rotate fan 112 to generate air flow. In some examples, motor 110 is configured to rotate fan 112 at maximum of 37,500 revolutions per minute (RPM). However, motor 110 may be configured to rotate fan 112 at a maximum of 50,000 RPM, 75, 000 RPM, or 100,000 RPM. Fan 112 may rotate to generate airflow that is outputted by blower 104. Motor 110 may be coupled to control system 106, which may control motor 110. In some examples, fan 112 is configured to provide a maximum of 1,000 liters per minute (LPM). In some examples, fan 112 is configured to rotate at greater than 37,500 RPMs and greater than 1,000 LPMs.

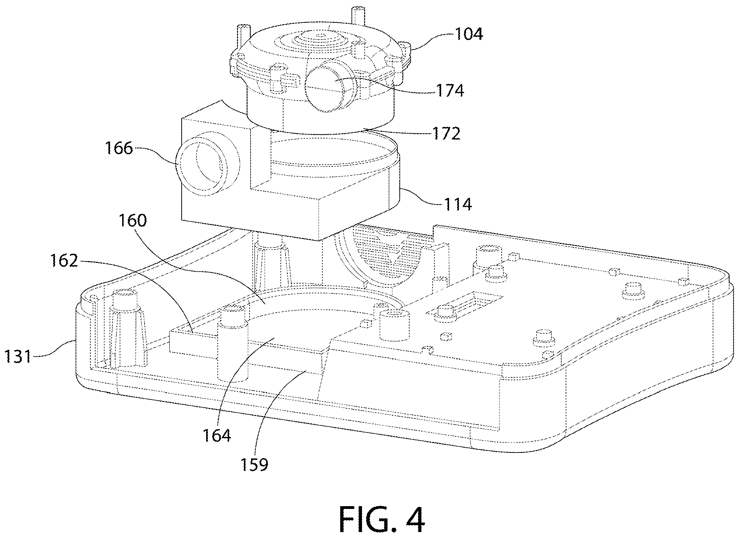

Referring to Figs. 4-9, blower 104 may be disposed within enclosure 114. Blower 104 may include blower outlet 174 and blower inlet 172. Blower inlet 172 may be configured to receive air and blower outlet 174 may be configured to blow air out from blower 104. Enclosure 114 may have a maximum length of approximately 91 mm, a maximum width of approximately 55 mm, and a maximum height of approximately 39 mm. However, enclosure 114 may have a maximum length of approximately 50 mm to approximately 250 mm, approximately 75 mm to approximately 225 mm, approximately 100 mm to approximately 200 mm, or approximately 125 mm to approximately 175 mm, a width of approximately 20 mm to approximately 200 mm, approximately 50 mm to approximately 150 mm, or approximately 75 mm to approximately 125 mm, and a thickness of approximately 25 mm to approximately 100 mm, approximately 30 mm to approximately 75 mm, or approximately 40 mm to approximately 100 mm.

Enclosure 114 may be sized and shaped to receive blower 104. In some examples, enclosure 114 is comprised of two halves that surround blower 104. However, enclosure 114 may be a unitary piece. For example, enclosure 114 may be configured to receive blower 104 such that blower 104 is disposed within enclosure 114. Enclosure 114 may be disposed within housing 132 such that when blower 104 is disclosed within enclosure 114, enclosure 114 is disposed within housing 132. Enclosure 114 being a unitary piece and configured to secure blower 104 within housing 132 allows for the reduction of components and material needed to manufacture ventilator system 100. Enclosure 114 may include inflow 169, which may pull air into blower 104 such that the air is received by blower 104.

In some examples, enclosure 114 is received by receptacle 159. Receptacle 159 may be sized and shaped to receive enclosure 114. In some examples, receptacle 159 is integrally formed with rear panel 131 of housing 132. For example, receptacle 159 may be integrally formed with rear panel 131 such that back wall 164 is the back wall of receptacle 159. However, receptacle 159 may be a separate component from back wall 164 and housing 132. Receptacle 159 may include recess 160. Enclosure 114 may be configured to be disposed within recess 160 of receptacle 159. Receptacle 159 may be integrally formed with housing 132 and enclosure 114 and recess 160 may assist in aligning blower 104 within housing 132 such that calibration and adjustment of the position of blower 104 is not required.

In some examples, blower 104 is disposed within enclosure 114, which is disposed within recess 160. However, enclosure 114 may be disposed within front panel 129. Blower 104 and enclosure 114 may be secured within recess 160 to create a substantially airtight seal around enclosure 114 and blower 104. For example, blower 104 may be disposed within enclosure 114, which is disposed within recess 160, and enclosure 114 may be configured to create an airtight seal with back wall 164 and recess lip 162 of recess 160 to allow air to flow from enclosure inlet 166 to blower inlet 172. In some examples, air flows from enclosure inlet 166 to blower inlet 172 without substantially leaking into housing 132 or buildup due to recess 160 creating an airtight seal around enclosure 114 and enclosure 114 creating an airtight tight seal around blower 104 thereby allowing air to flow easily into blower inlet 172. In some examples, the airtight seal around enclosure 114 and blower 104 results in less than 1% of air or gas leaking out of blower 104 and/or enclosure 114 into the rest of ventilator 102. However, the airtight seal around enclosure 114 and blower 104 may result in air or gas leakage of approximately 0.01% to approximately 5%, approximately 0.1% to approximately 4%, approximately 1% to approximately 3%, less than 5%, less than 4%, less than 3%, less than 2%, less than 1%, less than 0.5%, less than 0.1%, or less than 0.01%.

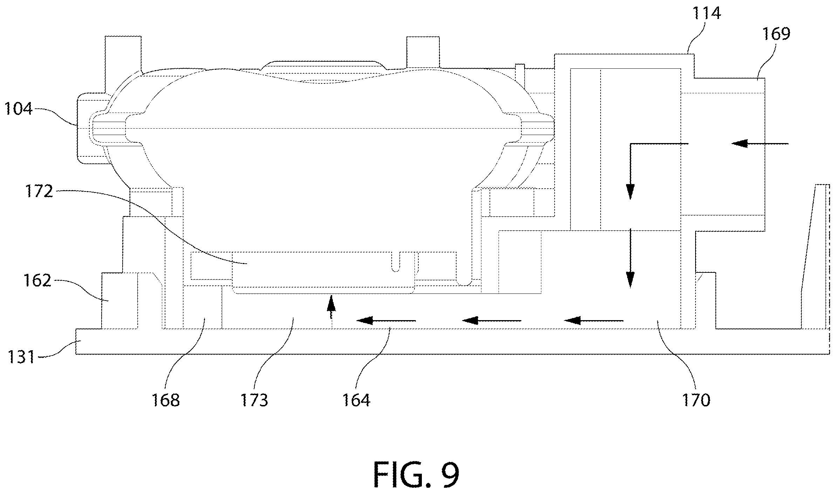

Referring to Fig. 9, air may flow into inflow 169 of enclosure 114. The air may then flow into interior space 170, which is formed between back wall 164 and blower 104. Blower 104 being disposed within enclosure 114, which is disposed in recess 160, creates an airtight seal between blower inlet 172 and back wall 164. This allows air to flow from inflow 169, through interior space 170, and into blower inlet 172 with minimal to no leakage of air.

In some examples, enclosure 114 may be additively manufactured. For example, enclosure 114 may be 3D printed. In alternative examples, enclosure 114 may be manufactured using a CNC machine or via injection molding. In some examples, enclosure 114 is comprised of Polycarbonate. However, enclosure 114 may be comprised of acrylonitrile butadiene styrene (ABS), polyoxymethylene (POM), aliphatic polyamides (PPA), polyphenylsulfone (PPSU), polyetherimide (PEI), or polypropylene (PP). In practice, use of enclosure 114 reduces the cost of the device as it decreases the amounts of components necessary, allowing for high-volume manufacturing. In some examples, enclosure 114 enables the precise location of all or portions of blower 104 within ventilator 102.

Referring to Figs. 4-9, blower 104 may be disposed within enclosure 114 such that blower inlet 172 is disposed within aperture 168. In some examples, an O-ring or gasket may be used between blower 104 and enclosure 114 to secure blower inlet 172 within aperture 168 and to create a substantially airtight seal between blower 104 and enclosure 114. Enclosure 114 may be hollow and may include interior space 170, which may be in communication with enclosure inlet 166 of enclosure 114. Enclosure 114 may be sized and configured to fit within recess 160 of rear panel 131 of housing 132. Enclosure 114 may be secured within recess 160 such that recess lip 162 surrounds the entirety of enclosure 114. In some examples, an O-ring or gasket may be disposed between recess lip 162 and enclosure 114. The O-ring or gasket may be configured to ensure that enclosure 114 is secured within recess 160 and that there is an airtight seal between enclosure 114, recess lip 162, and back wall 164 when blower 104 is secured within enclosure 114, and enclosure 114 is secured within recess 160.

Referring to Fig. 4-9, enclosure 114 may include enclosure inlet 166 which may be in communication with interior space 170. For example, as denoted by the arrows in Fig. 9, enclosure inlet 166 may be configured to receive air and allow air to flow into interior space 170. However, enclosure 114 may include other openings or holes to allow air to enter blower 104. For example, enclosure 114 may include one or more openings or holes to allow for leaking of excess air or gas, such as oxygen, in addition to pulling air into blower 104. Interior space 170 may be in communication with blower inlet 172 when blower 104 is disposed within enclosure 114. In some examples, when enclosure 114 is disposed within recess 160 and blower 104 is disposed within enclosure 114, ventilator system 100 may include gap or space 173 between blower inlet 172 and back wall 164 to allow blower inlet 172 to be in communication with interior space 170 and enclosure inlet 166. Blower 104 may be disposed within enclosure 114 such that blower inlet 172 is securely disposed within aperture 168. In practice, enclosure 114 may be disposed within recess 160 and blower 104 may be disposed within enclosure 114 such that blower inlet 172 is disposed within aperture 168 adjacent back wall 164 and a substantially airtight seal is created between blower inlet 172, recess lip 162, back wall 164, and interior space 170 to allow air to flow from enclosure inlet 166 to blower inlet 172.

In some examples, a portion of housing 132 comprises the back wall of enclosure 114. For example, housing 132 may comprise a portion of enclosure 114 such that housing 132 and enclosure 114 create a pathway for air flow from blower 104. Housing 132 and enclosure 114 may be configured to create a pneumatic/airflow pathway allowing for air to flow through ventilator 102.

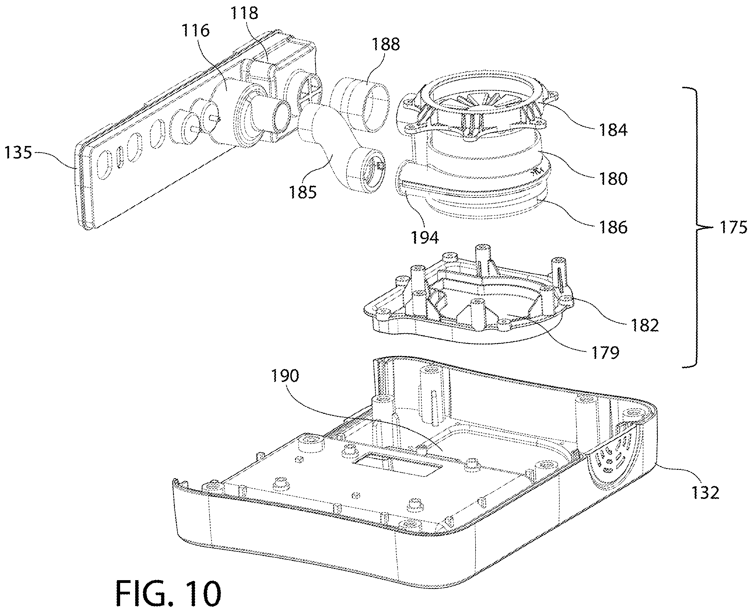

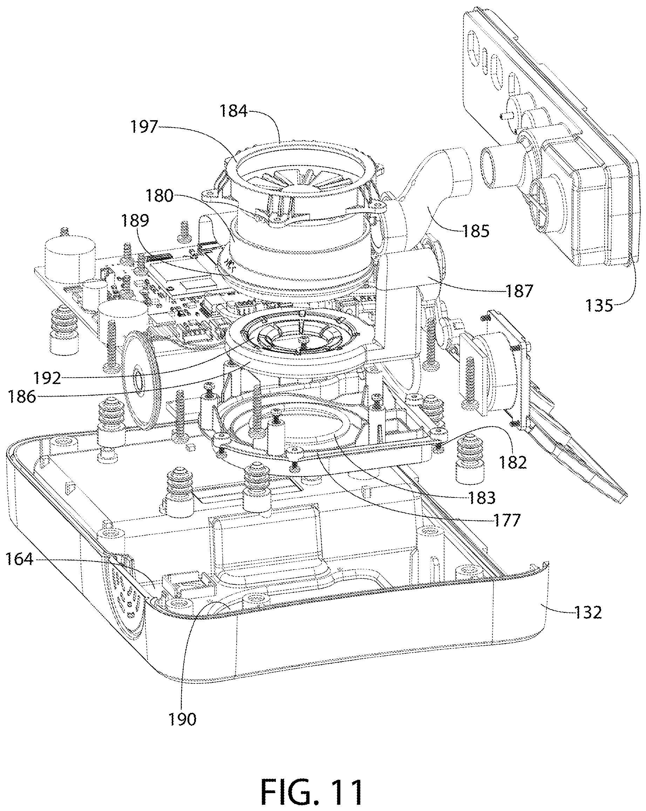

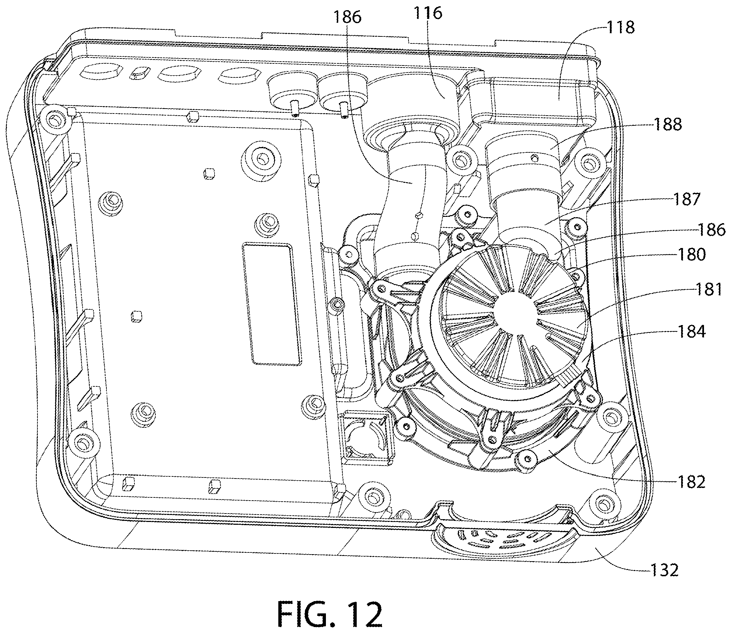

Referring to Figs. 10-12, ventilator 102 may include fan assembly 175. Similar to blower 104 and enclosure 114, fan assembly 175 may be configured to allow air to flow into ventilator 102 via inlet 118 from a gas source or ambient air and be pumped out via outlet 116 with minimal to no leakage or buildup of air. Fan assembly 175 may be configured to be modular. For example, fan assembly 175 may include blower 180, securing element 184, sealing element 186, and receptacle 182. Fan assembly 175 may be modular such that it is comprised of multiple components thereby allowing each component to be easily replaced without replacing the entirety of fan assembly 175. Fan assembly 175 may be coupled to housing 132. For example, housing 132 may include cutout 190 configured to receive fan assembly 175. In some examples, back wall 164 of housing 132 includes cutout 190 and cutout 190 is sized and shaped to receive receptacle 182. Receptacle 182 may be configured to couple to back wall 164 such that a portion of receptacle 182 is disposed within cutout 190. Receptacle 182 may be coupled to back wall 164 via fasteners. However, receptacle 182 may be coupled to back wall 164 via adhesives, magnets, or any other type of coupling mechanism. Receptacle 182 may include gasket 183, which may assist receptacle 182 with forming an airtight seal with blower 180 and sealing element 186. In some examples, receptacle 182 and sealing element 186 coupled together may be substantially the same as enclosure 114. The receptacle 182 includes a recess 177. Recess 177 may be sized and shaped to receive blower 180 and sealing element 186. For example, recess 177 may be configured to surround a substantial portion of sealing element 186. In some examples, the entirety of sealing aperture 192 is disposed within recess 177.

In some examples, sealing element 186 is coupled to receptacle 182 and blower 180. For example, sealing element 186 and blower 180 may be coupled to receptacle 182 such that blower 180 and sealing element 186 are at least partially disposed within recess 177. Sealing element 186 may include inlet portion 187, which may couple to inlet 118. In some examples, inlet portion 187 is coupled to inlet 118 via inlet adapter 188. In some examples, inlet adapter 188 is a modular component of fan assembly 175 and is configured to be easily swapped out and replaced. However, inlet adapter 188 may alternatively be integrally formed with inlet portion 187.

Sealing element 186 may be coupled to blower 180. Sealing element 186 may include sealing aperture 192. The sealing element 186 is coupled to blower inlet 189. Sealing element 186 may be coupled to blower 180 such that a portion or all of blower inlet 189 abuts or is disposed within sealing aperture 192. Sealing element 186 may assist with forming an airtight seal between blower inlet 189 and receptacle 182. Sealing element 186 forming an airtight seal may allow air to enter ventilator 102 via inlet 118 and travel through receptacle 182 and into blower inlet 189 of blower 180 via sealing aperture 192.

Blower 180 may be substantially the same as blower 104, but blower 180 may include heatsink 181. Heatsink 181 may be configured to dissipate heat generated by blower 180 and/or fan assembly 175. Incoming air (e.g., air entering blower 180 via blower inlet 189) may be pushed/forced out by blower 180 through blower outlet 194. Blower 180 may be secured to sealing element 186, which is secured to receptacle 182, via securing element 184. Securing element 184 may include securing aperture 197 and blower 180 may be received by securing element 184 such that blower 180 is disposed within securing aperture 197. Securing element 184 may couple to receptacle 182.

In some examples, securing element 184 is coupled to receptacle 182 such that blower 180 and sealing element 186 are disposed between securing element 184 and receptacle 182. Blower 180 and sealing element 186 being disposed between securing element 184 and receptacle 182 allows for a tight seal between blower 180, sealing element 186, and receptacle 182. For example, blower 180 may be coupled to sealing element 186, then blower 180 and sealing element 186 may be disposed with receptacle 182. Securing element 184 may be placed on blower 180 and coupled to receptacle 182. Tightening and securing of securing element 184 to receptacle 182 results in compression of blower 180 and sealing element 186 against receptacle 182 to create an airtight seal between blower 180, sealing element 186, and receptacle 182. Forming an airtight seal between blower 180, sealing element 186, and receptacle 182 maximizes the amount of air the is able to travel from inlet 118 to outlet 116.

In some examples, when blower 180 and sealing element 186 are disposed within receptacle 182, gasket 183 may be received by sealing aperture 192. For example, disposing sealing element 186 within receptacle 182 may result in aligning gasket 183 within sealing aperture 192 such that gasket 183 is at least partially disposed within sealing aperture 192. In some examples, gasket 183 may be disposed around the circumference of sealing aperture 192 to secure sealing element 186 in place within receptacle 182. Gasket 183 being disposed around the circumference of sealing aperture 192 prevent air from leaking out of receptacle 182 and sealing element 186 and maximizing the air entering blower inlet 189. In some examples, sealing element 186 includes a plurality of apertures disposed proximate blower inlet 189 when sealing element 186 is coupled to blower 180. The plurality of apertures may allow air flow from sealing element 186 and into blower inlet 189. For example, air may flow from inlet 118 through inlet portion 187 and out of the plurality of apertures such that the air can then flow into blower inlet 189.

In some examples, blower outlet 194 is coupled to outlet adapter 185. Outlet adapter 185 may be an S-shaped tube coupling blower 180 to outlet 116. In some examples, outlet adapter 185 is comprised of silicone. Outlet adapter 185 may integrally formed with blower outlet 194 or may be removably coupled to blower outlet 194. Inlet adapter 188 and outlet adapter 185 may assist in fan assembly 175 being modular. For example, damage to or obstruction of inlet adapter 188 and/or outlet adapter 185 may not require disassembly of fan assembly 175. This is because fan assembly 175 is modular thereby allowing inlet adapter 188 and/or outlet adapter 185 to be easily replaced without having to remove the entirety of fan assembly 175 from ventilator 102.

Similar to Fig. 9, fan assembly 175 of Figs. 10-12 allows air or gas (e.g., oxygen) to travel from inlet 118 to outlet 116. Air and gas may be used interchangeably herein. Air may enter fan assembly 175 from inlet 118 through inlet adapter 188 and into sealing element 186 via inlet portion 187. Air may then travel into blower inlet 189 with no or minimal leakage due to the tight seal between sealing element 186 and receptacle 182, with the assistance of gasket 183. Further, air may then flow easily into blower inlet 189 thereby reducing buildup of air. The air may then be forced/pumped out of blower 180 at an increased velocity via blower outlet 194.

In some examples, air or gas may flow from inlet 118 into sealing element 186. The air or gas may flow into interior space 179 formed between receptacle 182 and blower inlet 189. In some examples, interior space 179 is the area within sealing aperture 192. Blower 180 being disposed within sealing element 186, which is disposed within receptacle 182, creates an airtight seal between blower inlet 189 and receptacle 182. This allows air to flow from inlet portion 187, through interior space 179, and into blower inlet 189 with minimal to no leakage of air.

In some examples, ventilator 102 is configured to allow a small amount of air to leak to prevent accumulation of air within enclosure 114. For example, due to fire hazard concerns, enclosure 114 may prevent the buildup of air/gas within enclosure 114 by allowing a small amount of air/gas within enclosure 114 to leak out. In some examples, ventilator 102 may be configured to limit the amount of oxygen accumulated within enclosure 114 and/or housing 132. For example, enclosure 114 may include a sensor to measure the percentage of oxygen within enclosure 114 and/or housing 132 and may vent out the oxygen when above a pre-determined amount. For example, blower 104 of ventilator 102 may be configured to vent or leak out air/oxygen when the sensor determines that there is more than 15%, 20%, 25%, 30%, 35%, 40%, 45%, or 50% oxygen within enclosure 114 and/or housing 132. In some examples, ventilator 102 is configured to continuously vent or leak out air/oxygen to keep the amount of oxygen within enclosure 114 and/or housing 132 at or below 15%, 20%, 25%, 30%, 35%, 40%, 45%, or 50%.

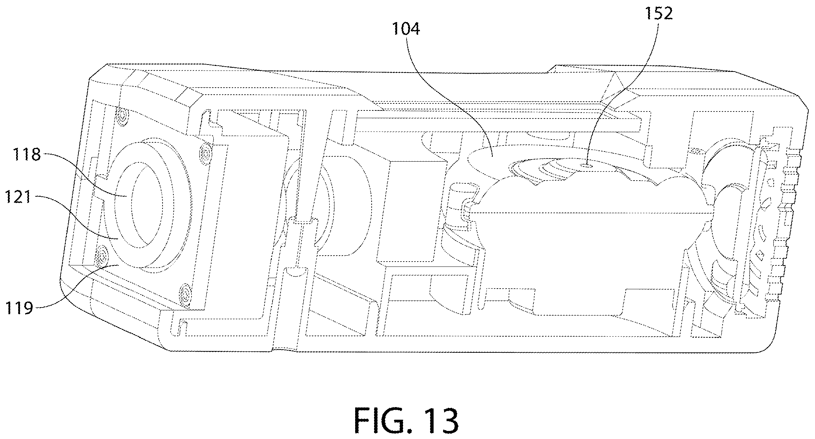

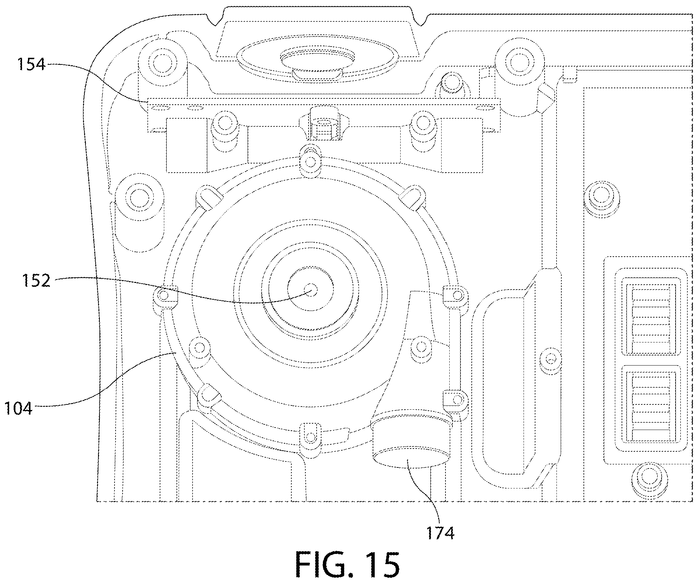

Referring to Figs. 13-16B, ventilator 102 may include exhaust opening 152 disposed on blower 104. For example, blower 104 may include exhaust opening 152, which may be configured to expel air that is disposed within blower 104. In some examples, exhaust opening 152 is configured to allow blower 104 to expel air, such as hot air, resulting in blower 104 cooling down and preventing overheating. In some examples, blower 104 leaks air, which builds up within enclosure 114 and/or housing 132. Exhaust opening 152 may be configured to allow blower 104 to pull in the leaked air to reduce the buildup of air within ventilator 102. In some examples, the leaked air is delivered to the patient. In some examples, enclosure 114 is used to direct the air out of blower 104 directly. In other examples, the exhaust air is run through side-stream oxygen sensor 154 before being vented out of housing 132 of ventilator 102.

In some examples, ventilator 102 includes exhaust conduit 156. Exhaust conduit 156 may be disposed on or within blower 104 and may be configured to provide a pathway for air/exhaust to exit blower 104 thereby preventing buildup of air within blower 104 and preventing overheating of blower 104. In some examples, ventilator 102 includes exhaust manifold 158, which includes exhaust inlet 161 and exhaust outlet 163. Exhaust manifold 158 may be communicatively coupled to exhaust conduit 156 and may be configured to provide a pathway for air to exit ventilator 102. Air may flow through exhaust manifold 158 to exhaust port 165, which is configured to provide a pathway for air to flow outside of ventilator 102.

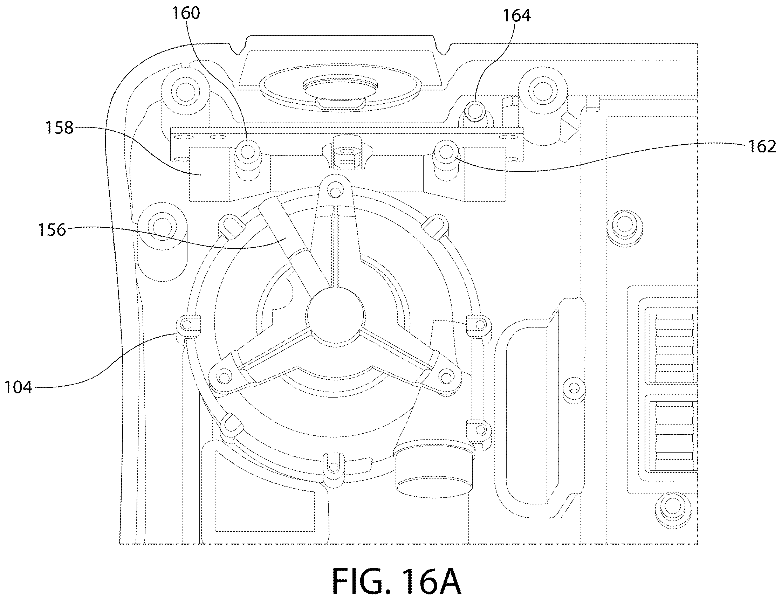

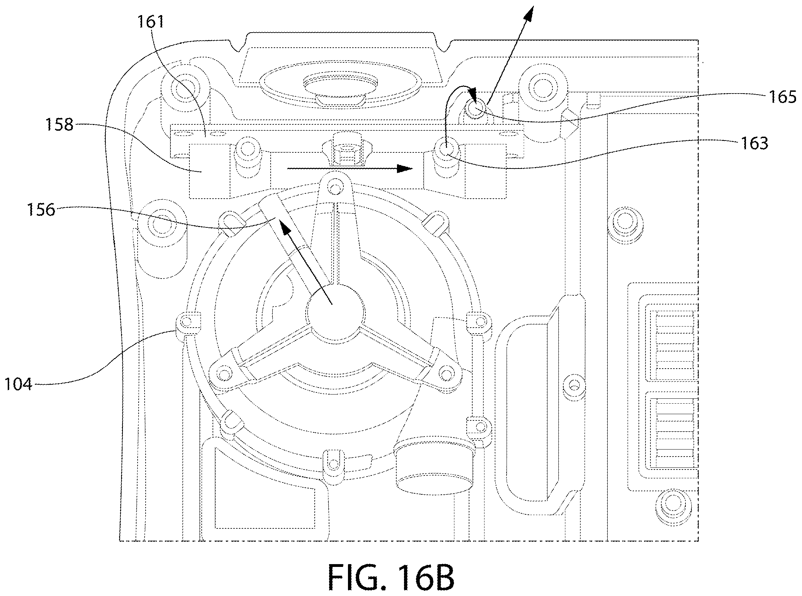

Referring to Fig. 16B, the flow of air from exhaust conduit 156 to exhaust port 165 is illustrated. In practice, air may flow from within blower 104 through exhaust conduit 156 to exhaust inlet 161 of exhaust manifold 158. Air may then exit exhaust manifold 158 via exhaust outlet 163 and may enter exhaust port 165, which may direct air to outside of ventilator 102.

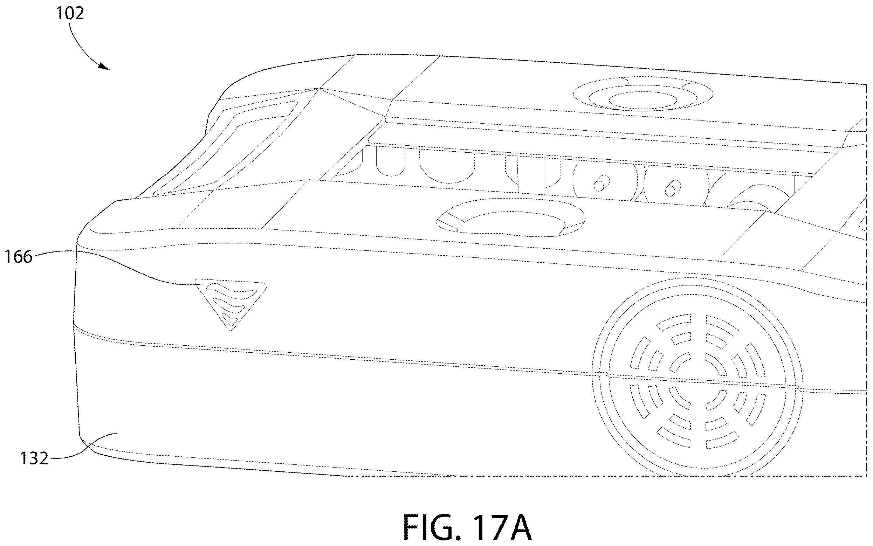

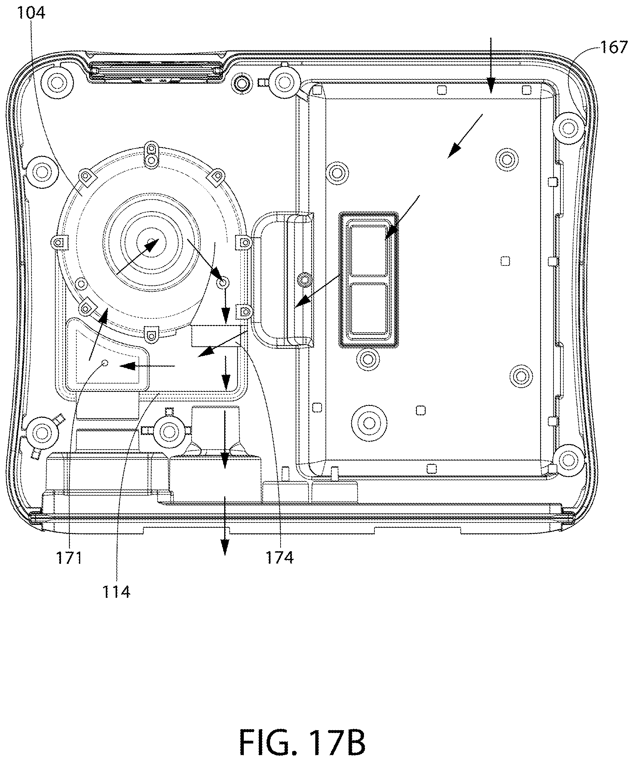

Referring to Figs. 17A and 17B, housing 132 may include opening 167 and enclosure 114 may include enclosure opening 171. Opening 167 may be disposed on housing 132 and may be configured to allow for venting of air, such as oxygen. In one example, opening 167 and enclosure opening 171 may utilize negative pressure generated by blower 104 to draw air in through opening 167 and enclosure opening 171 and out of blower outlet 174. Fig. 17B shows the air pathway from opening 167, through enclosure opening 171, and out of outlet 116. In some examples, opening 167 and enclosure opening 171 each assist with ventilating ventilator 102 and removal of accumulated gas, such as oxygen. This is crucial especially when there is a case of undetectable leak of gas, such as oxygen.

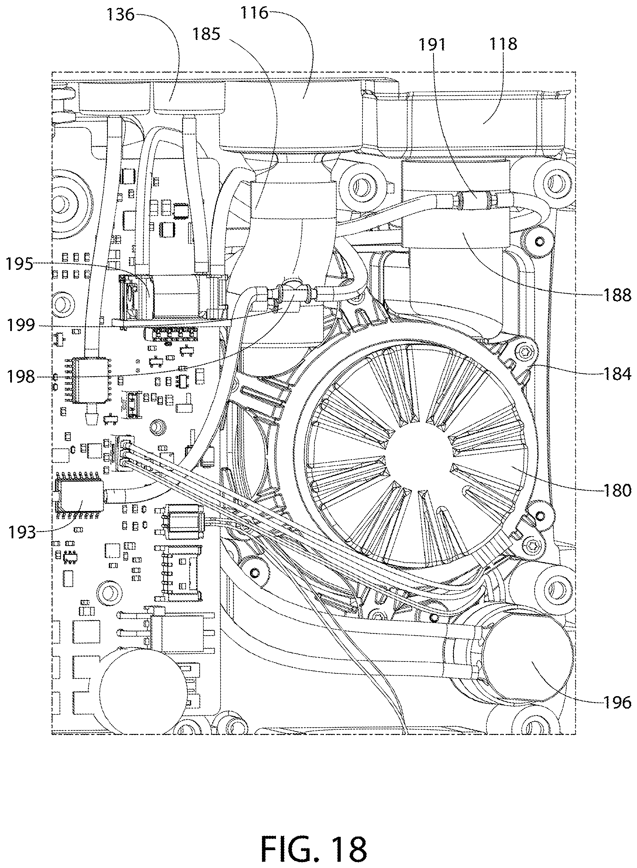

Referring to Fig. 18, outlet adapter 185 may include connector 198. Connector 198 may be configured to couple outlet adapter 185 to sensor 193 via one or more air tubes configured to allow for the flow of air or gas. Connector 198 may allow air or gas to flow from blower 180 through connector 198 to sensor 193. In some examples, connector 198 is a T-connector configured to couple outlet adapter 185, sensor 193, and valve 195 together via one or more tubes. However, connector 198 may be a valve such as a solenoid valve, rotary valve, a linear valve, a plug valve, or a ball valve. Sensor 193 may be a pressure sensor, a gas sensor, or any other type of sensor desired. For example, sensor 193 may be a pressure sensor and may be configured to measure the pressure of air or gas from blower 180 and through outlet adapter 185 and outlet 116. Connector 198 may further couple outlet adapter 185 to valve 195. In some examples, connector 198 allows air or gas to flow between outlet adapter 185, sensor 193, and valve 195.

Valve 195 may be coupled to outlet adapter 185 and inlet adapter 188. Valve 195 may further be coupled to control line port 136. Valve 195 may be a solenoid valve. However, valve 195 may be a valve such as a solenoid valve, rotary valve, a linear valve, a plug valve, or a ball valve. In some examples, connector 198 provides air from outlet adapter 185 to control line 616 via valve 195. For example, valve 195 may be coupled to control line port 136 via an air tube, which is in communication with control line 616. As discussed in further detail below, valve 195 being coupled to control line 616 may control exhale valve 208. For example, valve 195 is opened, air flows from outlet adapter 185 through control line 616. The resulting force from the air flow results in exhale valve 208 closing and remaining closed. In some examples, the pressure of air within control line 616 builds up. In order to relieve the built up pressure of air in control line 616, valve 195 may allow air to flow into inlet adapter 188 via connector 191. The air flowing into inlet adapter 188 may alleviate the pressure built up in control line 616. In some examples, valve 195 is configured to allow air to flow from control line 616 to inlet adapter 188 to prevent build up of air pressure within control line 616.

Outlet adapter 185 may also include connector 199. Connector 199 may couple outlet adapter 185 to sensor 196 via an air tube. Connector 199 may be an elbow joint configured to couple outlet adapter 185 to sensor 196. However, connector 199 may be a valve such as a solenoid valve, rotary valve, a linear valve, a plug valve, or a ball valve. In some examples, sensor 196 is a gas sensor. For example, sensor 196 may be an oxygen sensor configured to measure the amount of oxygen of the air or gas within through outlet adapter 185. Sensor 196 may be a galvanic oxygen sensor, an ultrasonic oxygen sensor, or any other type of oxygen sensor. In some examples, sensor 193 and sensor 196 are different sensors. For example, sensor 193 may be a pressure sensor and sensor 196 may be a gas sensor. In some examples, connector 198 only couples outlet adapter 185 to sensor 196, and sensor 196 may be both a gas and pressure sensor

In some examples representing the invention, sensor 196 is also coupled to inlet adapter 188. For example, sensor 196 may be coupled to inlet adapter at connector 191 via an air tube. Air or gas then flows from outlet adapter 185 to sensor 196 then to inlet adapter 188. For example, inlet adapter 188 may also be coupled to sensor 196 via an air tube. Air or gas from outlet adapter 185 flows to sensor 196 and is then discharged to inlet adapter 188. In some examples, sensor 196 is configured to receive air from outlet adapter 185 via connector 199, determine the concentration of a certain gas in the air from outlet adapter 185, and then direct the air to inlet adapter 188 via connector 191. In practice, sensor 196 is configured to measure air or gas from outlet adapter 185 and discharge the air or gas to inlet adapter 188. For example, air may flow from outlet adapter 185 through connector 199 to sensor 196. Sensor 196 may measure a concentration (e.g., oxygen) within the air then direct the air to inlet adapter 188 via connector 191 to ensure that air is not wasted when it flows from outlet adapter 185 to sensor 196. This configuration increases efficiency of ventilator 102 as no air is wasted during sampling and measuring of gas concentration via sensor 196.

In some examples, each of outlet adapter 185 and inlet adapter 188 are coupled to valve 195. Valve 195 may be configured to control the flow of air to and from outlet adapter 185 and inlet adapter 188. For example, valve 195 may be coupled to connector 198 of outlet adapter 185 and/or connector 191 of inlet adapter 188 to control the flow of air in and out of outlet adapter 185 and/or inlet adapter 188.

In some examples, connector 198, connector 191, valve 195 and sensor 193 assist fan assembly 175 and ventilator 102 prevent buildup of air or gas (e.g., oxygen). For example, connector 198, connector 191, valve 195, and sensor 193 may be configured to monitor the pressure and control the path of air throughout fan assembly 175 to prevent leakage of air. Further, connector 198, connector 191, valve 195 and sensor 193 may assist in maximizing the efficiency of ventilator 102 by allow air from outlet adapter 185 that is sent for testing/monitoring (e.g., via sensor 196) to be recirculated back to inlet adapter 188 to be used by blower 180 and outputted to the patient via outlet 116.

Referring to Figs. 1A-1B, ventilator 102 may include control system 106. Control system 106 may be a microcontroller, a peripheral interface controller (PIC), a system on a chip (SoC), or a processor. In some examples, control system 106 is a lower power controller. For example, control system 106 may be a lower power controller coupled to a power supply such that control system 106 is configured to run for extended period of time (e.g., several years). Control system 106 may be coupled to one or more components of ventilator system 100. In some examples, control system 106 is coupled to blower 104 to control motor 110, which controls fan 112. In some examples, control system 106 controls the volume of gas delivered to a patient by attenuating the speed of fan 112. For example, controls system 106 may attenuate the power delivered to motor 110, thereby be decreasing the speed of fan 112 to reach a target amount of gas delivered to a patient through breathing circuit 200. Control system 106 may include writing device 113, which may be configured to write information to transmitting devices 117, such as radiofrequency identification (RFID) chips/tags. In some examples, control system 106 is coupled to power supply 108. However, control system 106 may be coupled to its own power supply.

In some examples, writing device 113 is disposed within ventilator 102. However, writing device 113 may be disposed outside of ventilator 102 and may be an external device. Writing device 113 may be disposed within, on, or outside of ventilator 102 and may wirelessly communicate with transmitting device 117. In some examples, writing device 113 is configured to wirelessly write information to transmitting devices 117. Writing device 113 may be coupled to control system 106 and may be stored anywhere within ventilator 102. Writing device 113 may further be coupled to memory 115, which may be coupled to control system 106.

In some examples, transmitting device 117 is stored within ventilator 102 and is communicatively coupled to control system 106. However, transmitting device 117 may be disposed on or near housing 132 of ventilator 102 and may be configured to wirelessly communicate with control system 106. For example, transmitting device 117 may be coupled to the exterior surface of housing 132 and may wirelessly receive information from control system 106. Transmitting device 117 may be a storage device configured to wirelessly transmit information, such as a wireless transmitting device. For example, transmitting device 117 may include one or more of an RFID chip/tag, a near-field communication chip, a Bluetooth transmitter, a digital barcode, or a Wi-Fi module. In some examples, transmitting device 117 only transmits information upon request. However, transmitting device 117 may be configured to transmit information automatically and/ or autonomously without intervention by a user or external device. Transmitting device 117 may be configured for low-power consumption and may be configured to receive power only from an external source. However, transmitting device 117 may be powered by power supply 108 or its own power supply.

Control system 106 may receive information associated with, for example, the status of ventilator system 100 and store the information in memory 115 or directly to transmitting device 117. Writing device 113 may access memory 115 and may write the information stored within memory 115 to transmitting device 117. In some examples, memory 115 includes transmitting device 117. Memory 115 may include, for example, random access memory (RAM), a hard disk drive and/or a removable storage drive, such as a floppy disk drive, a magnetic tape drive, an optical disk drive, or a wireless device, such as an RFID tag. Memory 115 may include other similar means for allowing computer programs or other instructions to be loaded into ventilator system 100. For example, memory 115 may include a removable memory chip (such as an EPROM, or PROM, or flash memory) and associated socket, and other removable storage units and interfaces which allow software and data to be transferred from a removable storage unit to ventilator system 100. In some examples, memory 115 is a non-volatile memory. In some examples, memory 115 is configured for low-power consumption or configured to receive power only from an external source.

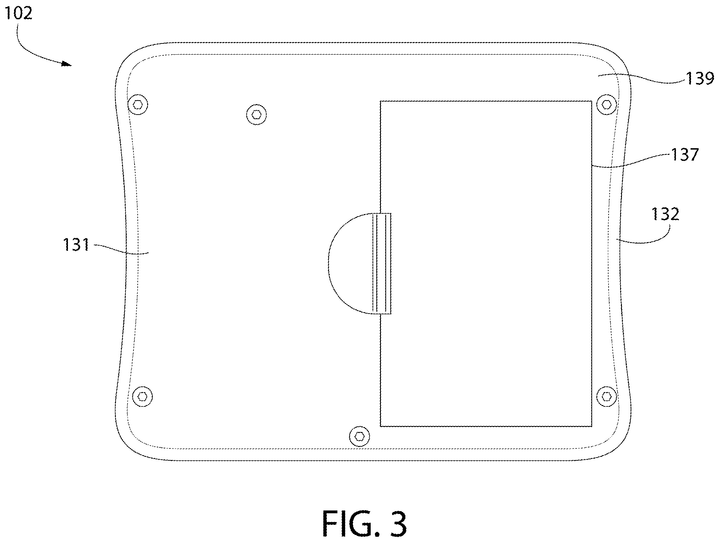

In some examples, control system 106 is coupled to power supply 108, which may be configured provide power to the various components of ventilator system 100. For example, control system 106 may be configured to route power from power supply 108 to motor 110 of blower 104. Power supply 108 may be disposed within ventilator 102. Power supply 108 may include one or more of an internal rechargeable battery, a removable rechargeable battery, and a removable non-rechargeable battery. As shown in Fig. 3, ventilator 102 may be configured to receive a battery pack via battery storage 137. In some examples, a user may place a removable rechargeable battery and/or a removable non-rechargeable battery within battery storage 137. In some examples, power supply 108 may be coupled to a power source (not shown) via a power adapter. Power supply 108 may control the voltage and current from a power source to control system 106.

In some examples, ventilator system 100 is configured to administer a status check or a self-test to ensure that all components are working properly and that there are not any malfunctions. In some examples, control system 106 is configured to test the various components of ventilator system 100 to determine the functional status of, for example, blower 104, power supply 108, writing device 113, memory 115, transmitting device 117, and control system 106, in addition to reporting the operational status of ventilator system 100. For example, control system 106 may be configured to receive information from memory 115 regarding any corrupted cores, from blower 104 regarding an occlusion of fan 112, from outlet 116 or inlet 118 regarding occlusions, from power supply 108 regarding improper voltages, or any other information necessary to ensure that ventilator 102 is functioning properly.

In some examples, ventilator 102 of ventilator system 100 is configured to administer a status check, store the results of the status check, and then power down. In some examples, ventilator 102 is configured to perform a self-test while ventilator 102 is in storage or otherwise not in active use (e.g., in a powered down state). The results of the status check may be stored on memory 115, which may be configured to transmit the results without receiving power from ventilator 102. For example, ventilator 102 may power on, administer a status check, store the results of the status check on transmitting device 117 and/or memory 115, and then power down. Transmitting device 117 may be configured to transmit the results only when interrogated by an external source. The external source may be a receiving or reading device that provides power to transmitting device 117 enabling transmitting device 117 to transmit the results. This allows ventilator 102 to conserve power as it does not need to power on to transmit the results of the status check and enables ventilator 102 to provide results at any time upon interrogation by a user.

In some examples, ventilator 102 includes inlet 118 and outlet 116. Inlet 118 may be disposed on one of sidewalls 130 of housing 132 and allow for air to flow from the external environment (ambient air) or an air source, such as a reservoir of gas (O₂), to blower 104. For example, blower 104 may be configured to pull in air from inlet 118 and push the air out through outlet 116. In some examples, ventilator 102 relies on blower 104 to provide air and does not require compressed air to operate. In some examples, blower 104 is coupled to outlet 116, which is disposed on an outer periphery of housing 132. For example, outlet 116 may be disposed on sidewall 130 of housing 132. Outlet 116 may be cylindrical in shape and hollow. In some examples, outlet 116 couples blower 104 to breathing circuit 200 to patient interface 300. For example, outlet 116 may be configured to allow air to flow from blower 104 of ventilator 102 through breathing circuit 200 to patient interface 300. In some examples, outlet 116 is a valve that may open or close to control the airflow from blower 104 to breathing circuit 200. In some examples, blower 104 includes blower inlet 172. Blower inlet 172 may be in communication with inlet 118 and may be configured to pull air into blower 104, which is then pushed out of blower 104 at a higher flow rate by fan 112.

Referring to Figs. 1A-1B and 19-20, ventilator system 100 may include breathing circuit 200. Breathing circuit 200 may be coupled to ventilator 102. In some examples, breathing circuit 200 may be disposed between ventilator 102 and patient interface 300. For example, breathing circuit 200 may be configured to couple ventilator 102 to patient interface 300. Breathing circuit 200 may be configured to receive air from ventilator 102. Breathing circuit 200 may include tube 202, exhale valve 208, flow sensor 210, and patient filter 212. Tube 202 may include first end 204 and second end 206. First end 204 may be coupled to ventilator 102 and second end 206 may be coupled to patient interface 300. In some examples, tube 202 is a cylindrical lumen configured to allow airflow from ventilator 102 to patient interface 300. Tube 202 may be configured to include exhale valve 208, flow sensor 210, and patient filter 212. Exhale valve 208 disposed on or within tube 202 and may be configured to open on exhalation of the patient using ventilator system 100 to allow air to flow out of the patient. Exhale valve 208 may be closed during inhalation such that air does not exist ventilator system 100, thereby increasing efficiency. For example, exhale valve 208 may be closed during inhalation to ensure that the proper amount and flow of air reaches patient interface 300. In some examples, exhale valve 208 being closed during inhalation reduces the amount of air leaked or wasted (e.g., not used by the patient) within ventilator system 100.

In some examples, breathing circuit 200 does not include an active control or anti-asphyxiation valve that are traditionally used to prevent/reduce asphyxiation in the patient. In traditional ventilators, asphyxiation valves are used when there is device failure, and the patient needs to breathe spontaneously by allowing gas from the external atmosphere into the breathing circuit. Further, traditional ventilators utilize an anti-asphyxiation/check valve to maintain PEEP, while allowing the patient to breathe spontaneously if the device fails or set incorrectly. In practice, breathing circuit 200 allows ventilator system 100 to maintain pressure with positive pressure from blower 104, instead of a valve. In some examples, ventilator system 100 maintains PEEP by driving motor 110 of blower 104 on exhalation. Breathing circuit 200 may be configured to not utilize an anti-asphyxiation/check valve by including a non-obstructed pathway between the patient and an air source. This allows the patient to breathe even when there is failure to ventilator system 100. Breathing circuit 200 allows for a clear path for the patient to inhale and inspire ambient air without obstructions even when motor 110 and/or blower 104 fails.

In some examples, exhale valve 208 is controlled by control system 106 to control the exhalation of the patient. In another example, exhale valve 208 is controlled based on the exhalation of the patient. In yet another example, exhale valve 208 is controlled by both control system 106 and the exhalation of the patient. Exhale valve 208 may be configured to allow for a specific respiration rate but may be opened by the exhalation of the patient as well. For example, for a respiration rate of 12 (one breath every five seconds), exhale valve 208 may open every five seconds and may also open more than every five seconds if the patient is breathing at different rate.

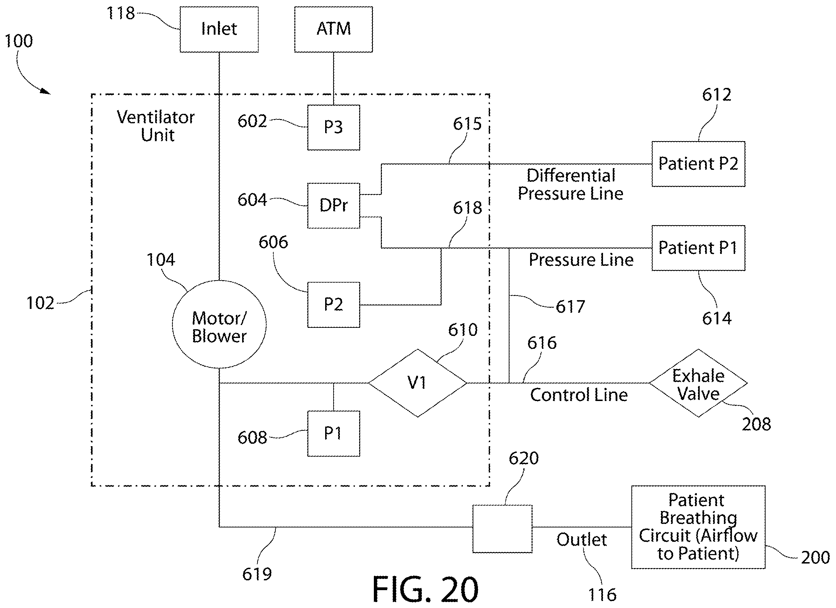

Referring to Fig. 20, exhale valve 208 may be driven by control line 616, which may be commutatively coupled to ventilator 102 via control line port 136. Exhale valve 208 may be driven by control line 616 using valve 610 and/or valve 195. In some examples, valve 610 is the same as valve 195 of Fig. 18. However, valve 610 and valve 195 may be different. Valve 610 may be a solenoid valve and may be housed inside ventilator 102. In some examples, when valve 610 is opened by control system 106, air flows from blower 104 through control line 616. The resulting force from the air flow results in exhale valve 208 closing and remaining closed. In some examples, the pressure reading at pressure sensor 608 allows ventilator system 100 to validate whether valve 610 has correctly opened or closed. In some examples, pressure sensor 608 determines whether valve 610 is correctly opened by reading atmospheric pressure and whether valve 610 is closed by reading pressure of control line 616. In some examples, pressure sensor 606 is configured to infer that the valves are in an open/closed state from the patient pressure reading. In recognizing a standard patient exhalation (rapid pressure drop), ventilator 102 can determine that the valve has correctly opened.

In practice, on inhalation, ventilator system 100 may be configured such that valve 610 is opened, resulting in exhale valve 208 closing such that no gas/air leaves ventilator system 100 via exhale valve 208. On exhalation, valve 610 may be closed, allowing exhale valve 208 to be opened since no positive pressure is being exerted on exhale valve 610 and the gas exhaled by the patient may be vented into the atmosphere. In some examples, PEEP is maintained after exhalation by driving motor 110 at a lower speed to prevent backflow into ventilator system 100, which would cause CO2 rebreathing. In some examples, the patient exhalation passes through a breathing filter so that internal components and atmosphere are protected from potential microbial contamination, dust, dirt and other particulate.

In some examples, ventilator system 100 is configured to allow the patient to breathe during failure of ventilator system 100 by using blower 104 and valve 610, as discussed above. In this configuration, if there is a fault that results in critical failure of ventilator system 100 and motor 110/blower 104 stops, exhale valve 208 may be opened. Opening of exhale valve 208 during failure allows the patient to freely be able to breathe. If exhale valve 208 is stuck closed or there is an issue with control system 106, the patient will still be able to breathe through breathing circuit 200 since there is no check valve between the patient's airway and inlet 118 preventing backflow. If there is a failure that causes motor 110 and/or fan 112 to get stuck in an active position (inhale state), exhalation would still be possible because exhale valve 208 has a maximum pressure limit that the patient would be able to overcome through their force of breathing. In some examples, fan 112 may be a non-occlusive fan configured to allow air flow even when motor 110 and/or fan 112 becomes stuck or fails. In some examples, ventilator system 100 includes a blow-off/check valve configured to release pressure within breathing circuit 200 when a pre-determined pressure is reached.

In some examples, ventilator system 100 determines inspiratory pressure using pressure line 618 that is connected to breathing circuit 200 near the patient. The pressure reading of pressure sensor 606 at pressure line 618 may be used to determine the measured PIP (max inspiratory pressure per breath cycle), PEEP, and to generate pressure graphs. In some examples, a flow meter external to ventilator 102 may determine airflow and may be connected to ventilator 102 via It is connected to the device using a flow line. The data from the flow meter may be used to measure flow into and out of the patient, and thus to calculate Tidal Volume and other patient monitoring parameters such as minute volume amongst other things. In some examples, ventilator system 100 includes a pressure sensor to determine the atmospheric pressure and is configured to calibrate delivery of air to the patient via breathing circuit 200 accordingly.

In some examples, ventilator system 100 may be configured to obtain a differential pressure reading at differential pressure sensor 604. The differential pressure reading may be used to measure flow. Since flow is proportional to the pressure drop of a gas/liquid moving over a resistance in a pipe, ventilator 102 can determine the air flow to and from the patient. With some intentional resistance in breathing circuit 200, and a differential pressure measurement from one side of the resistance to the other (and proper calibration), ventilator 102 can determine the flow in breathing circuit 200. In some examples, ventilator system 100 includes differential pressure line 615, which provides air to patient sensor 612, which may be a pressure sensor.

In some examples, breathing circuit 200 includes flow sensor 210, which may be disposed on or within tube 202. Flow sensor 210 may be configured to sense the flow of air within breathing circuit 200. For example, flow sensor 210 may detect the rate and amount of air flowing through tube 202. In some examples, flow sensor 210 is coupled to control system 106 to provide feedback to ventilator system 100. For example, flow sensor 210 may provide information to control system 106, which may change the parameters of blower 104 based on the information.

Breathing circuit 200 may further include patient filter 212, which may be disposed proximate second end 206 of tube 202. For example, patient filter 212 may be disposed on or within tube 202 proximate second 206 and adjacent to patient interface 300. Patient filter 212 may be configured to filter out particles within air. For example, patient filter 212 may filter out particles and airborne viruses to protect the patient using ventilator system 100.

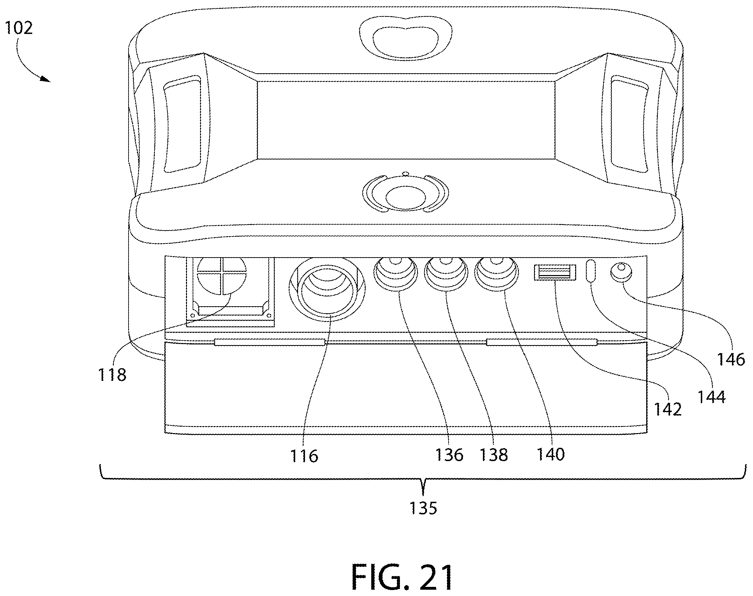

Referring to Fig. 21, ventilator 102 may further included various inputs for coupling ventilator 102 to other components of ventilator system 100. In addition to inlet 118 and outlet 116, ventilator 102 may include control line port 136, pressure line port 138, differential pressure tube port 140, flow sensor port 142, data communication port 144, and power port 146. Control line port 136 may be used to couple exhale valve 208 and ventilator 102. For example, exhale valve 208 may be coupled to ventilator 102 at control line port 136 such that ventilator 102 can control the opening and closing of exhale valve 208. Pressure line port 138 and differential pressure tube port 140 may be used to couple one or more pressure sensors to ventilator 102. Flow sensor port 142 may be used to couple flow sensor 210 to ventilator 102. For example, flow sensor 210 may be coupled to ventilator 102 at flow sensor port 142 such that ventilator 102 can receive information from flow sensor 210. Data communication port 144 may be used to couple ventilator 102 to an electronic device such as a computer system, a mobile device, a server, etc. Power port 146 may be used to couple ventilator 102 to a power source. For example, power port 146 may be configured to couple power supply 108 to a power source to provide power to ventilator 102 through power supply 108.

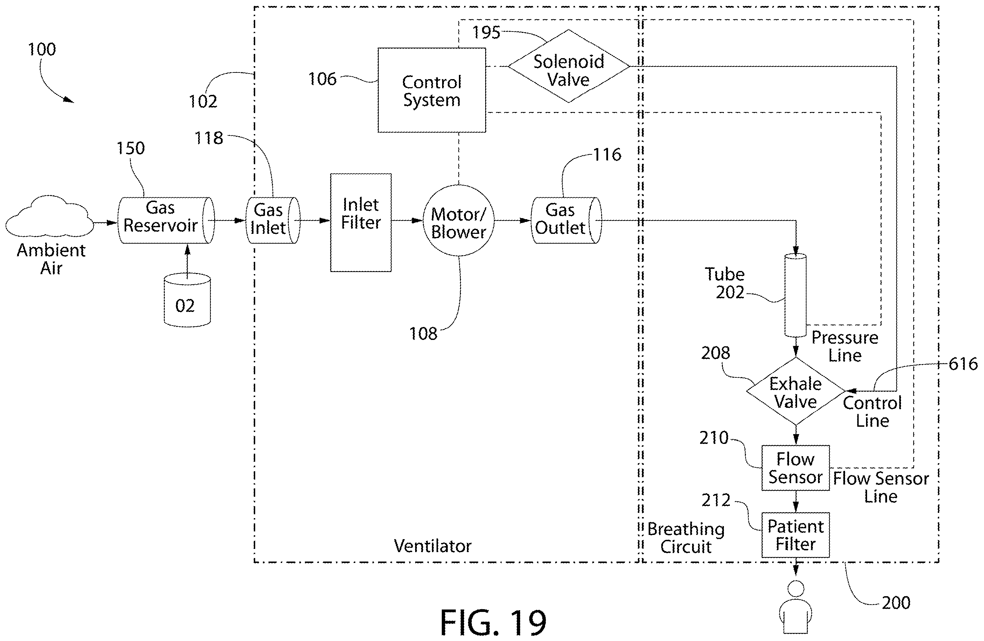

Referring to Fig. 19, air (e.g., ambient air) and/or gas (e.g., oxygen) may both enter gas reservoir 150 and mix together. Gas from gas reservoir 150 may enter ventilator 102 through inlet 118. Inlet 118 may include a filter to prevent external debris from entering ventilator 102. The gas is then channeled through an air pathway housed in ventilator 102, and into breathing circuit 200 through outlet 116. Once the gas from ventilator 102 is within breathing circuit 200, the flow of the gas is measured by flow sensor 210, and the air passes through patient filter 212 before entering the patient via patient interface 300.

Referring to Fig. 13, ventilator 102 may include port plate 119. Port plate 119 may a portion of housing 132 that protects port inputs 135. Port inputs 135 may include one or more of inlet 118, outlet 116, control line port 136, pressure line port 138, differential pressure tube port 140, flow sensor port 142, data communication port 144, and power port 146. Port plate 119 may be configured to prevent debris from entering the ports of ventilator 102. In some examples, port plate 119 includes one or more filters to filter air/gas entering through various inlets of ventilator 102. Port plate 119 may be hingedly coupled to housing 132. In some examples, port plate 119 is a separate component from housing 132 and may be slidably received by housing 132 adjacent to the ports of ventilator 102. For example, port plate 119 may be molded to housing 132 and may be manufactured via injection molding.

Inlet 118 may include cover or door 121 disposed over inlet 118. Cover 121 may be configured to allow inlet 118 to be connected to air/gas source, such as an oxygen source. Inlet 118 may also include cover 121 to prevent connection of the wrong connector to inlet 118. For example, inlet 118 may include a specialized cover configured to allow only for reservoirs of only certain gases or fluids to flow into inlet 118. In some examples, cover 121 prevents inadvertent connection of breathing circuit 200 to the wrong connection. In some examples, a user would have to actively remove cover 121 from inlet 118 to allow connection of an air/gas source to inlet 118. Cover 121 may be coupled to port plate 119. For example, cover 121 may be hingedly coupled to port plate 119 to allow for covering of inlet 118. In some examples, cover 121 may allow ambient air to flow into inlet 118 without removing cover 121 from inlet 118. In some examples, cover 121 includes special markings to indicate the sources of air/gas that can be coupled to inlet 118. In some examples, a special tool is required to remove cover 121 from inlet 118 to prevent inadvertent connection to inlet 118. In some examples, cover 121 includes a sensor to only allow removal from inlet 118 when certain gases are detected. Cover 121 may also be configured to prevent debris from entering inlet 118.

In some examples, port plate 119 includes a testing cap configured to allow for the testing of airflow and pneumatic system 104 of ventilator 102. The testing cap may be configured to disposed over port plate 119 and allow air coming from outlet 116 of fan 112 to flow through the testing cap into a pressure sensor disposed on port plate 119 or the testing cap. For example, the testing cap may include a recess that allows air to flow form outlet 116 to the pressure sensor to determine the pressure of air provided by pneumatic system 104. The recess of the testing cap may allow for air to be channeled from outlet 116 to the pressure sensor, which may be disposed on port plate 119. For example, the testing cap may allow for testing of pneumatic system 104 when ventilator 102 is in storage. The testing cap may be configured to ensure the integrity of pressure sensors of ventilator 102 in addition to providing additional protection to port plate 119 and outlet 116. In some examples, the recess of testing cap allows air to flow from outlet 116 to other sensors disposed on port plate 119 and/or within the testing cap. The testing cap may be hingedly coupled to port plate 119 or housing 132 and may be configured to be completely removable from ventilator 102.

Referring to Fig. 20, ventilator system 100 may include connection line 617. Connection line 617 may couple pressure line 618 and control line 616 and may be configured to be an open or closed state. For example, in an open state, air flows from control line 616 to pressure line 618 and in a closed state, air does not flow between control line 616 and pressure line 618. In some examples, pressure line 618 is blocked from providing air to patient sensor 614 and control line 616 is blocked from providing air to exhale valve 208 along air to flow through connection line 617. Ventilator system 100 may include exhale line 619, which provides an air path to outlet 116. In some examples, ventilator system 100 includes outlet valve 620 configured to be closed, which stops the flow of air within exhale line 619, thereby preventing air from reaching outlet 116.

In some examples, blower 104 is ramped up to test the functionality of blower 104 via pressure sensor 608. For example, by ramping up blower 104 and blocking outputs of pressure line 618, control line 616, and exhale line 619, pressure is built up via connection line 617, thereby allowing pressure sensor 608 to receive a pressure reading. In some examples, blower 104 includes a tachometer configured to provide RPM (revolutions per minute) data. In some examples, the RPM data is correlated with pressure readings, such that a specific RPM equates to a specific pressure reading at pressure sensor 608. In other words, a pressure reading at pressure sensor 608 may be dependent on the RPM data provided by the tachometer. For example, pressure sensor 608 may output a pressure value based on the RPM data provided by the tachometer. Given a mismatch in the expected pressure based on the RPM data and the pressure reading at pressure sensor 608, an error may have occurred in ventilator system 100, such as blower 104 or with pressure sensor 608.

In some examples, the functionality of each of pressure sensor 608, pressure sensor 606, and differential pressure sensor 604 are tested by ramping up blower 104. For example, by ramping up blower 104, having valve 610 open, and outlet valve 620 closed, air may travel from blower 104 to pressure sensor 608 and through valve 310 and connection line 617 to pressure sensor 606 and differential pressure sensor 604. Each of pressure sensor 608, pressure sensor 606, and differential pressure sensor 604 may provide the same pressure reading when valve 610 is opened, outlet valve 620 is closed, and connection line 617 is an opened state. In some examples, the pressure reading at pressure sensor 608 and pressure sensor 606 is dependent on the RPM data provided the tachometer disposed within blower 104.

In some examples, ventilator system 100 is configured to test the functionality of valve 610. For example, ventilator system 100 may open valve 610 and vent out and release the air within blower 104 to the atmosphere, which should result in the pressure reading at pressure sensor 608 to decrease since the pressure is no longer built up. Ventilator system 100 may close valve 610, which thereby blocks the air path to exhale valve 208 via control line 616. Closing valve 610 results in the pressure reading at pressure sensor 608 increasing and the pressure reading at pressure sensor 606 decreasing to, for example, atmospheric pressure. In some examples, the pressure reading at pressure sensor 606 is substantially the same as the pressure reading at pressure sensor 608, which confirms that both pressure sensor 606 and pressure sensor 608 are operating correctly. In some examples, opening valve 610 results in the venting of air to atmosphere, which results in a decrease in the pressure reading at pressure sensor 606. In some examples, opening valve 610 allows for pressure line 618 to connect with control line 616 via connection line 617, which allows ventilator system 100 to assess pressure sensor 606 and differential pressure sensor 604.

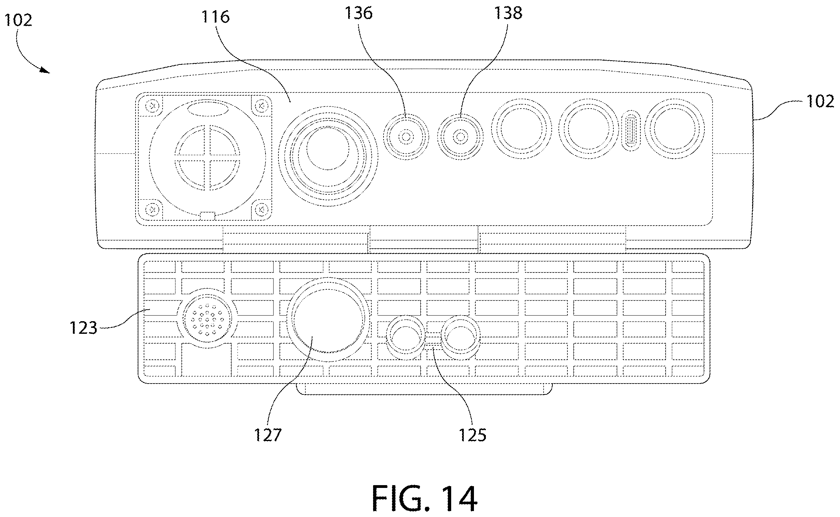

Referring to Fig. 14, ventilator 102 may include door 123, which is configured to cover one or more ports of ventilator 102. Door 123 may be coupled to housing 132. For example, door 123 may be hingedly coupled to housing 132 such that door 123 is able to cover and uncover one or more ports disposed on ventilator 102. However, door 123 may be removably coupled to housing 132 or coupled to housing 132 via an adhesive, fasteners, magnets, or any other method of coupling door 123 to housing 132. In some examples, door 123 includes connection channel 125, which is configured to connect control line port 136 and pressure line port 138. For example, connection channel 125 may form connection line 617 allowing pressure line 618 to couple to control line 616. In some examples, door 123 include plug 127, which is configured to plug outlet 116. Plug 127 may be outlet valve 620, configured to stop the flow of air within exhale line 619, and prevent air from reaching outlet 116.

It will be appreciated by those skilled in the art that changes could be made to the examples shown and described above. It is understood, therefore, that this invention is not limited to the examples shown and described, but it is intended to cover modifications within the scope of the present invention as defined by the claims. For example, specific features of the exemplary examples may or may not be part of the claimed invention and various features of the disclosed examples may be combined. Unless specifically set forth herein, the terms "a", "an" and "the" are not limited to one element but instead should be read as meaning "at least one".

It is to be understood that at least some of the figures and descriptions of the invention have been simplified to focus on elements that are relevant for a clear understanding of the invention, while eliminating, for purposes of clarity, other elements that those of ordinary skill in the art will appreciate may also comprise a portion of the invention. However, because such elements are well known in the art, and because they do not necessarily facilitate a better understanding of the invention, a description of such elements is not provided herein.

## Claims

1. A ventilator (102) comprising:
a housing (132) having a front panel (129), and a rear panel (131) opposite the front panel and configured to couple to the front panel;
a receptacle (159; 182) coupled to the rear panel (131) of the housing (132), the receptacle having a recess (160; 177);
a sealing element (186) disposed within the recess (160; 177), the sealing element having an inlet (187) and an aperture (192); and
a blower (104; 180) having a blower outlet (174; 194) and a blower inlet (172; 189), the blower inlet coupled to the sealing element such that the blower inlet at least partially abuts the aperture (192),
wherein the receptacle (159; 182) and the sealing element (186) comprise an airflow pathway configured to allow for flow of air from the inlet (187) of the sealing element (186) into the blower inlet (174; 194) and out of the blower outlet,
wherein the blower outlet (174; 194) and the inlet (187) are coupled to a sensor (196), the sensor configured to receive air from the blower outlet (174; 194) and direct the air to the inlet (187).

2. The ventilator (102) of claim 1, further comprising a further sensor configured to measure the percentage of gas within the housing (132) or an enclosure (114) in which the blower (104; 180) is disposed, wherein the blower (104; 180) is configured to vent gas through an exhaust opening (152) when the gas reaches a predetermined amount as determined by the further sensor.

3. The ventilator (102) of claim 1, further comprising a further sensor configured to measure the percentage of gas within the within the housing (132) or an enclosure (114) in which the blower (104; 180) is disposed, wherein the blower is configured to continuously vent gas through an exhaust opening to keep the gas at or below a predetermined level as determined by the further sensor.

4. The ventilator (102) of claim 2 or claim 3, wherein the gas is oxygen, and the predetermined amount or predetermined level is at least 25%.

5. The ventilator (102) of claim 1, further comprising:
a gap (173) between the blower inlet (172; 189) and the receptacle (159; 182) to allow air to flow into blower inlet.

6. The ventilator (102) of claim 1, wherein the recess (160; 177) includes a recess lip (162) and the sealing element (186) is surrounded by the recess lip.

7. The ventilator (102) of claim 1, wherein the receptacle (159; 182) is:
integrally formed with the rear panel (131) of the housing (132); or
disposed within a cutout (190) on the rear panel (131) of the housing (132).

8. The ventilator (102) of claim 1, wherein the recess (160; 177) is sized and shaped to receive the sealing element (186).

9. The ventilator (102) of claim 1, wherein the aperture (192) of the sealing element (186) is sized and shaped to receive at least a portion of the blower inlet (172; 189).

10. The ventilator (102) of claim 1, wherein the sealing element (186) is an additively manufactured enclosure.

11. The ventilator (102) of claim 1, further comprising:
a securing element (184) coupled to the receptacle (159; 182), the securing element configured to receive the blower (104; 180) and secure the blower and sealing element (186) within the recess (160; 177) of the receptacle.

12. The ventilator (102) of claim 1, wherein the blower (104; 180), the sealing element (186), and the receptacle (159; 182) are coupled together to form a substantially airtight seal.

13. The ventilator (102) of claim 1, further comprising:
one or more ports (118, 116, 136, 138, 140, 142, 144, 146) disposed on the housing (132); and either:
a port plate (119) configured to at least partially cover the one or more ports (118, 116, 136, 138, 140, 142, 144, 146), wherein the port plate is removable from the housing (132); or
a door (123) configured to at least partially cover the one or more ports (116, 136, 138), wherein the door is hingedly coupled to the housing (132) and includes at least one of a plug (127) and connection channel (125).

14. The ventilator (102) of claim 1, further comprising:
a breathing circuit (200) coupled to the sealing element (186), the breathing circuit having an exhale valve (208) configured to open and close based on air flow generated by the blower (104; 180).

## Patentansprüche

1. Beatmungsgerät (102), umfassend:
ein Gehäuse (132), das eine Frontplatte (129) und eine Rückplatte (131) aufweist, die der Frontplatte entgegengesetzt und dazu konfiguriert ist, mit der Frontplatte gekoppelt zu werden;
eine Aufnahme (159; 182), die mit der Rückwand (131) des Gehäuses (132) verbunden ist, wobei die Aufnahme eine Aussparung (160; 177) aufweist;
ein Dichtungselement (186), das in der Aussparung (160; 177) angeordnet ist, wobei das Dichtungselement einen Einlass (187) und eine Öffnung (192) aufweist; und
ein Gebläse (104; 180), das einen Gebläseauslass (174; 194) und einen Gebläseeinlass (172; 189) aufweist, wobei der Gebläseeinlass so mit dem Dichtungselement gekoppelt ist, dass der Gebläseeinlass mindestens teilweise an der Öffnung (192) anliegt,
wobei die Aufnahme (159; 182) und das Dichtungselement (186) einen Luftströmungsweg umfassen, der dazu konfiguriert ist, einen Luftstrom vom Einlass (187) des Dichtungselements (186) in den Gebläseeinlass (174; 194) und aus dem Gebläseauslass heraus zu ermöglichen,
wobei der Gebläseauslass (174; 194) und der Einlass (187) mit einem Sensor (196) gekoppelt sind, wobei der Sensor dazu konfiguriert ist, Luft vom Gebläseauslass (174; 194) zu empfangen und die Luft zum Einlass (187) zu leiten.

2. Beatmungsgerät (102) nach Anspruch 1, das weiter einen weiteren Sensor umfasst, der dazu konfiguriert ist, den Prozentsatz an Gas innerhalb des Gehäuses (132) oder eine Kapselung (114), in der das Gebläse (104; 180) angeordnet ist, zu messen, wobei das Gebläse (104; 180) dazu konfiguriert ist, Gas durch eine Auslassöffnung (152) abzulassen, wenn das Gas eine vorbestimmte Menge erreicht, wie durch den weiteren Sensor bestimmt.

3. Beatmungsgerät (102) nach Anspruch 1, das weiter einen weiteren Sensor umfasst, der dazu konfiguriert ist, den Prozentsatz an Gas innerhalb des Gehäuses (132) oder eine Kapselung (114), in der das Gebläse angeordnet ist, zu messen, wobei das Gebläse (104; 180) dazu konfiguriert ist, fortlaufend Gas durch eine Auslassöffnung abzulassen, um das Gas auf oder unter einem vorbestimmten Pegel zu halten, wie durch den weiteren Sensor bestimmt.

4. Beatmungsgerät (102) nach Anspruch 2 oder 3, wobei das Gas Sauerstoff ist und die vorgegebene Menge oder der vorgegebene Pegel mindestens 25 % beträgt.

5. Beatmungsgerät (102) nach Anspruch 1, weiter umfassend:
einen Spalt (173) zwischen dem Gebläseeinlass (172; 189) und der Aufnahme (159; 182), um Luft zu ermöglichen, in den Gebläseeinlass zu strömen.

6. Beatmungsgerät (102) nach Anspruch 1, wobei die Aussparung (160; 177) eine Aussparungslippe (162) beinhaltet und das Dichtungselement (186) von der Aussparungslippe umgeben ist.

7. Beatmungsgerät (102) nach Anspruch 1, wobei die Aufnahme (159; 182) Folgendes ist:
integral mit der Rückwand (131) des Gehäuses (132) gebildet; oder
in einem Ausschnitt (190) an der Rückwand (131) des Gehäuses (132) angeordnet.

8. Beatmungsgerät (102) nach Anspruch 1, wobei die Aussparung (160; 177) dazu bemessen und geformt ist, das Dichtungselement (186) aufzunehmen.

9. Beatmungsgerät (102) nach Anspruch 1, wobei die Öffnung (192) des Dichtungselements (186) dazu bemessen und geformt ist, mindestens einen Teil des Gebläseeinlasses (172; 189) aufzunehmen.

10. Beatmungsgerät (102) nach Anspruch 1, wobei das Dichtungselement (186) eine additiv gefertigte Kapselung ist.

11. Beatmungsgerät (102) nach Anspruch 1, weiter umfassend:
ein Sicherungselement (184), das mit der Aufnahme (159; 182) gekoppelt ist, wobei das Sicherungselement dazu konfiguriert ist, das Gebläse (104; 180) aufzunehmen und das Gebläse und das Dichtungselement (186) in der Aussparung (160; 177) der Aufnahme zu sichern.

12. Beatmungsgerät (102) nach Anspruch 1, wobei das Gebläse (104; 180), das Dichtungselement (186) und die Aufnahme (159; 182) miteinander gekoppelt sind, um eine im Wesentlichen luftdichte Dichtung zu bilden.

13. Beatmungsgerät (102) nach Anspruch 1, weiter umfassend:
einen oder mehrere Anschlüsse (118, 116, 136, 138, 140, 142, 144, 146), die am Gehäuse (132) angeordnet sind; und entweder:
eine Anschlussplatte (119), die dazu konfiguriert ist, den einen oder die mehreren Anschlüsse (118, 116, 136, 138, 140, 142, 144, 146) mindestens teilweise abzudecken, wobei die Anschlussplatte vom Gehäuse (132) abnehmbar ist; oder
eine Tür (123), die dazu konfiguriert ist, den einen oder die mehreren Anschlüsse (116, 136, 138) mindestens teilweise abzudecken, wobei die Tür scharnierartig mit dem Gehäuse (132) gekoppelt ist und mindestens eines von einem Stecker (127) und einem Verbindungskanal (125) beinhaltet.

14. Beatmungsgerät (102) nach Anspruch 1, weiter umfassend:
einen Atemkreislauf (200), der mit dem Dichtungselement (186) gekoppelt ist, wobei der Atemkreislauf ein Ausatemventil (208) aufweist, das dazu konfiguriert ist, sich basierend auf dem vom Gebläse (104; 180) erzeugten Luftstrom zu öffnen und zu schließen.

## Revendications

1. Ventilateur (102), comprenant :
un boîtier (132) comportant un panneau avant (129) et un panneau arrière (131) opposé au panneau avant et configuré pour se coupler au panneau avant ;
un réceptacle (159 ; 182) couplé au panneau arrière (131) du boîtier (132), le réceptacle présentant un évidement (160 ; 177) ;
un élément d'étanchéité (186) disposé à l'intérieur de l'évidement (160 ; 177), l'élément d'étanchéité présentant une entrée (187) et une ouverture (192) ; et
une soufflante (104; 180) comportant une sortie de soufflante (174; 194) et une entrée de soufflante (172 ; 189), l'entrée de soufflante étant couplée à l'élément d'étanchéité de telle sorte que l'entrée de soufflante vienne en butée au moins partiellement contre l'ouverture (192),
dans lequel le réceptacle (159 ; 182) et l'élément d'étanchéité (186) comprennent un trajet d'écoulement d'air configuré pour permettre l'écoulement de l'air depuis l'entrée (187) de l'élément d'étanchéité (186) dans l'entrée de soufflante (174 ; 194) et hors de la sortie de soufflante,
dans lequel la sortie de soufflante (174 ; 194) et l'entrée (187) sont couplées à un capteur (196), le capteur étant configuré pour recevoir de l'air de la sortie de soufflante (174 ; 194) et diriger l'air vers l'entrée (187).

2. Ventilateur (102) selon la revendication 1, comprenant en outre un autre capteur configuré pour mesurer le pourcentage de gaz à l'intérieur du boîtier (132) ou d'une enceinte (114) dans laquelle la soufflante (104; 180) est disposée, dans lequel la soufflante (104; 180) est configurée pour évacuer le gaz à travers une ouverture d'échappement (152) lorsque le gaz atteint une quantité prédéterminée telle que déterminée par l'autre capteur.

3. Ventilateur (102) selon la revendication 1, comprenant en outre un autre capteur configuré pour mesurer le pourcentage de gaz à l'intérieur du boîtier (132) ou d'une enceinte (114) dans laquelle la soufflante (104; 180) est disposée, dans lequel la soufflante est configurée pour évacuer en continu le gaz à travers une ouverture d'échappement pour maintenir le gaz à ou en dessous d'un niveau prédéterminé tel que déterminé par l'autre capteur.

4. Ventilateur (102) selon la revendication 2 ou la revendication 3, dans lequel le gaz est de l'oxygène, et la quantité prédéterminée ou le niveau prédéterminé est au moins égal à 25%.

5. Ventilateur (102) selon la revendication 1, comprenant en outre :
un espace (173) entre l'entrée de soufflante (172 ; 189) et le réceptacle (159 ; 182) pour permettre à l'air d'écouler dans l'entrée de soufflante.

6. Ventilateur (102) selon la revendication 1, dans lequel l'évidement (160 ; 177) comprend une lèvre d'évidement (162) et l'élément d'étanchéité (186) est entouré par la lèvre d'évidement.

7. Ventilateur (102) selon la revendication 1, dans lequel le réceptacle (159 ; 182) est :
formé d'un seul tenant avec le panneau arrière (131) du boîtier (132) ; ou
disposé dans une découpe (190) sur le panneau arrière (131) du boîtier (132).

8. Ventilateur (102) selon la revendication 1, dans lequel l'évidement (160 ; 177) est dimensionné et formé pour recevoir l'élément d'étanchéité (186).

9. Ventilateur (102) selon la revendication 1, dans lequel l'ouverture (192) de l'élément d'étanchéité (186) est dimensionnée et formée pour recevoir au moins une partie de l'entrée de soufflante (172 ; 189).

10. Ventilateur (102) selon la revendication 1, dans lequel l'élément d'étanchéité (186) est une enceinte fabriquée de manière additive.

11. Ventilateur (102) selon la revendication 1, comprenant en outre :
un élément de fixation (184) couplé au réceptacle (159 ; 182), l'élément de fixation étant configuré pour recevoir la soufflante (104 ; 180) et fixer la soufflante et l'élément d'étanchéité (186) dans l'évidement (160 ; 177) du réceptacle.

12. Ventilateur (102) selon la revendication 1, dans lequel la soufflante (104 ; 180), l'élément d'étanchéité (186) et le réceptacle (159 ; 182) sont couplés ensemble pour former un joint sensiblement étanche à l'air.

13. Ventilateur (102) selon la revendication 1, comprenant en outre :
un ou plusieurs orifices (118, 116, 136, 138, 140, 142, 144, 146) disposés sur le boîtier (132) ; et soit :
une plaque d'orifice (119) configurée pour recouvrir au moins partiellement les un ou plusieurs orifices (118, 116, 136, 138, 140, 142, 144, 146), dans lequel la plaque d'orifice est amovible par rapport au boîtier (132) ; soit
une porte (123) configurée pour recouvrir au moins partiellement les un ou plusieurs orifices (116, 136, 138), dans lequel la porte est couplée de manière articulée au boîtier (132) et comprenant au moins un parmi un bouchon (127) et un canal de connexion (125).

14. Ventilateur (102) selon la revendication 1, comprenant en outre :
un circuit respiratoire (200) couplé à l'élément d'étanchéité (186), le circuit respiratoire comportant une soupape d'expiration (208) configurée pour s'ouvrir et se fermer en fonction du flux d'air généré par la soufflante (104 ; 180).
